# EUROPÄISCHE PATENTSCHRIFT

(11) **EP 3 331 450 B1**
(45) Veröffentlichungstag und Bekanntmachung des Hinweises auf die Patenterteilung: **06.11.2019**
(21) Anmeldenummer: 16753867.7
(22) Anmeldetag: 03.08.2016
(51) Int. Cl.: A61B 10/00, A61B 5/20, A61B 5/00, G01N 33/487, G01N 33/493, A61B 5/145, G01N 33/483

(54) **VORRICHTUNG UND VERFAHREN ZUR MOBILEN ANALYSE VON EXKREMENTEN IN DER TOILETTE**
DEVICE AND METHOD FOR THE MOBILE ANALYSIS OF EXCREMENT IN A TOILET
DISPOSITIF ET PROCÉDÉ D'ANALYSE MOBILE D'EXCRÉMENTS DANS LES TOILETTES

(30) Priorität: 03.08.2015 DE 102015112678; 22.04.2016 DE 102016107486
(43) Veröffentlichungstag der Anmeldung: 13.06.2018
(73) Patentinhaber: MEDIPEE GMBH, 47445 Moers (DE)
(72) Erfinder: PROKOPP, Thomas, 53125 Bonn (DE)
(74) Vertreter: Farago-Schauer, Peter Andreas
(86) Internationale Anmeldenummer: PCT/EP2016/068553
(87) Internationale Veröffentlichungsnummer: WO 2017/021452

(56) Entgegenhaltungen:
- DE-A1- 3 402 488
- DE-T2- 60 126 448
- US-A- 4 466 445
- US-A- 5 625 911
- US-A1- 2005 261 605
- US-A1- 2008 312 556

## Beschreibung

Die Erfindung betrifft eine Vorrichtung und ein Verfahren zur Bestimmung physiologischer Daten durch Analyse menschlicher Exkremente in einer Toilette mittels wenigstens einem Indikator und/oder Sensor, durch Messung von Urin- und/oder Stuhlwerten.

Das Gesundheitswesen wird in zunehmendem Maße digitalisiert. Tragbare Minicomputer sind aus der Consumer-Welt von heute nicht mehr wegzudenken. Sie können eine Vielzahl von Daten erfassen und in Echtzeit analysieren, beispielsweise Vitalparameter wie Aktivitäts- und Ruhephasen, Blutzuckerspiegel, Gewicht, Körperfettwerte etc.. Die Geräte sind meist kabellos mit "mobilen Endgeräten" verbunden, um die Daten zu Gesundheits-, Ernährungs- und Aktivitätsstatus umgehend auswerten zu können. Dies bedeutet einen Quantensprung für Therapie und Diagnostik. Die Möglichkeit zur selbstständigen oder Fern-Überwachung von Körperdaten wird in Zukunft mehr und mehr kostenintensive Kontrollbesuche beim Arzt oder im Krankenhaus ersetzen.

Das große Marktpotenzial der Consumer-Produkte beruht zum einen auf einer immer älter werdenden Menschheit und immer größer werdenden gesundheitsbewussten Bevölkerungsgruppen und deren Bestreben, Vitalparameter zu erfassen. Die Daten werden meist präventiv für eine umfassende Beurteilung des Gesundheitszustands eingesetzt und damit auch für die Optimierung des eigenen Lebensstils. Zum anderen wird die Entwicklung durch Trends wie Frühstadiendiagnostik, "eHealth" und Telemedizin vorangetrieben.

Dem Gros dieser Analysen ist gemein, dass sie vornehmlich Werte äußerer Vitalparameter messen und dokumentieren. Vitalparameter aus dem Körperinneren oder sog. ,Laborwerte' bleiben aufgrund der schwierigeren Akquise von Proben und der aufwendigeren Analyse oft außen vor.
Dabei birgt die Analyse von Blut oder Exkrementen (Urin und Stuhl) eine weitaus umfangreichere Aussagekraft über den tatsächlichen Gesundheitszustand des Körpers, als lediglich die Analyse von Temperatur, Puls oder Blutdruck. Es können Hinweise auf mögliche Krankheiten bereits in Frühstadien gefunden werden, was wiederum die Chance auf eine Heilung erheblich steigert. Eine große Anzahl Menschen stirbt unnötigerweise an Krankheiten, die durch eine Frühdiagnose gut heilbar gewesen wäre.

Da die Analyse von Blut nach dem heutigen Stand der Technik im Entnahmeprozess immer invasiv ist, d.h. Entnahme mit einer Spritze oder Ritzen der äußeren Haut, sowie einen gewissen Hygienestandard voraussetzt, ist für den unbefangenen, täglichen oder wöchentlichen Gebrauch eine substantielle Hürde gegeben.

Hingegen werden bei allen Menschen Urin und Stuhl meist mehrmals am Tag ausgeschieden. Üblicherweise werden Defäkation und Miktion auf einer Toilette verrichtet.

Über Verfahren mit Reagenzien ist es möglich, sehr viele Werte wie beispielsweise pH-Wert, Proteine, Glukose, die Keton-Körper, Bilirubin, verborgenes Blut, Urobilinogen, Nitrat als auch Mikroben innerhalb kürzester Zeit zu messen. Dabei werden Reagenzien, Testpapiere und Enzyme benötigt, die oft an Tragegliedern angebracht sind (beispielsweise Teststreifen).

Die gängigste Methode, um den Urin zu untersuchen, ist ein Urin-Schnelltest. Dabei wird ein Teststreifen, auf dem sich kleine quadratische Farbfelder befinden, ein paar Sekunden in den Urin eingetaucht. Je nachdem, in welcher Konzentration die jeweilige Substanz vorhanden ist, verfärben sich die Felder des Teststreifens und können somit gemessen werden. Durch Urintests ist es möglich, Bestandteile, wie beispielsweise Glukose, Proteine, Hormone, Vitamine oder verborgenes Blut zu messen, um Hinweise auf einen möglicherweise vorliegenden Diabetes mellitus, einer Nephrose, einer Hepatitis odereiner anderen Entzündungserkrankung, einen Stein oder Tumor der Niere, der Blase oder der Harnröhre, bzw. eine Prostatitis zu finden. Es ist möglich, das Vorhandensein verschiedener Krankheiten in frühen Stadien zu entdecken, um die Gesundheit, durch das tägliche Aufzeichnen von Konzentrationen solcher Urinbestandteile, zu bewahren. Auch Schwangerschaftstests, Ovulationstests und teilweise der Konsum von Drogen lassen sich mittels Urintests durchführen. Urintests eignen sich darüber hinaus für die Überprüfung des Stoffwechselhaushalts bei Sportlern oder während einer Diät. Aber auch eine Bestimmung des Blutalkoholwertes kann über Messungen des Urins vorgenommen werden.

Der vom Körper aufgenommene Alkohol wird auf verschiedene Weise entsorgt: Bis zu 5 % wird unverändert abgeatmet. Ca. 2 % des getrunkenen Alkohols werden unverändert mit dem Urin ausgeschieden, 1 - 2 % Alkohol werden über die Haut ausgeschwitzt. Von dem im Körper verbleibenden Alkohol werden ca. 90-95 % über die Alkoholdehydrogenase katalysiert (ADH), in der Leber zunächst zu Acetaldehyd, dann katalysiert durch das Enzym Aldehyddehydrogenase (ALDH) zur Essigsäure aufoxidiert. Das Acetat wird über Citratcyclus und Atmungskette unter Energiegewinnung zu Wasser und Kohlendioxid abgebaut. Das Konzentrations-ZeitVerhalten von Blutalkohol ist sehr gut untersucht. Weil die Toxikokinetik des Ethanols bekannt ist, ist es sogar möglich, auf das Konsumverhalten der letzten Stunden zurückzurechnen. Andererseits wird Ethanol relativ schnell im Körper abgebaut. Der mit Abstand größte Teil des aufgenommenen Alkohols wird über die Essigsäure abgebaut, aber es gibt auch zahlreiche Nebenreaktionen: In einer nichtoxidativen Nebenreaktion (nur etwa 0,5 % der gesamten Ethanol-Eliminierung) wird der Alkohol mit Uridin-5'-diphospho-ß-glucuronsäure ("aktivierter" Glucuronsäure, UDP-D-Glucuronsäure) zu Ethylglucuronid (EtG) umgesetzt. Ethyl-Glucuronid wird ausschließlich aus Ethanol als direkter Alkohol-Metabolit gebildet. Weil keine endogenen EtG-Spiegel existieren, wird EtG somit zu einem hochspezifischen Marker für Alkoholkonsum, der bereits nach einmaligem Konsum einer geringen Menge im Serum nachweisbar ist. Die maximale EtG-Konzentration wird dabei 3-10 Stunden nach dem Konsum erreicht, die maximale Ausscheidungsrate nach ca. 3-5 Stunden. Die Halbwertszeit in der Anfangsphase beträgt 2-3 Stunden.
Katalysiert wird die Reaktion zum Ethyl-Glucuronid durch das Enzym UDP-Glucuronosyltransferase, das in verschiedenen Ausprägungen im menschlichen Metabolismus vorkommt. Für den Alkohol-Abbau sind im Wesentlichen zwei Typen relevant, die je nach genetischer Disposition im menschlichen Organismus aufzufinden sind: Ein schnell wirkender und ein langsam metabolisierender Typ. Eine genaue quantitative Bestimmung des konsumierten Alkohols aus der ermittelten Menge an EtG ist deshalb ohne die Kenntnis der genetischen Variante des Probanden schwer möglich. Ebenso können der Konsum-Zeitpunkt sowie die Art des Getränkes heute noch nicht bestimmt werden. Dessen ungeachtet besteht zumindest eine semiquantitative Relation zwischen der Konsum-Menge und der resultierenden EtG-Konzentration: Hohe EtG-Werte lassen eindeutig einen Rückschluss auf entsprechend hohe Alkohol-Konsumierung zu. Die Ausscheidung des Ethyl-Glucuronids erfolgt über die Nieren, wobei auch kleine Blutkonzentrationen noch im Urin aufkonzentriert werden und so nachweisbar sind. Untersuchungen zur Stabilität von Urinproben belegen, dass der EtG-Gehalt sich innerhalb von 4 Tagen um weniger als 10 % verringert. Im Vergleich zum Blutalkohol ist EtG bedeutend länger messbar; zwar ist eine Rückrechnung auf den ursprünglichen Blutalkoholgehalt nur schwer möglich, aber die Menge an gemessenem EtG lässt durchaus qualitative Rückschlüsse auf das Trinkverhalten zu.

Durch Zugriff auf zusätzliche Datenbestände einer Person (Größe, Gewicht, genetischen Herkunft etc.) lässt sich durch entsprechende Algorithmen eine sehr genaue Annäherungen an den tatsächlichen Blutalkoholgehalt aus dem Urin generieren. Diese Daten können beispielsweise durch mobile Endgeräte beigesteuert werden. Aber auch Vergleichshochrechnungen von Personen mit ähnlichen physiognomischen oder genetischen Merkmalen lassen sich dazu nutzen.

Durch Urintests lassen sich auch Diäten oder der Stoffwechsel, beispielsweise bei Sportlern, über die Messung der Ketonkörper kontrollieren. Ketonkörper sind beim Abbau von Fett entstehende Substanzen. Im engeren Sinn versteht man darunter das Azetoazetat, das Beta-Hydroxybutyrat und das Azeton. Ketonkörper sind Energielieferanten für viele Gewebe, auch für das Gehirn. Besonders im Hungerzustand wird Energie aus Ketonkörpern gewonnen. Beim Fettabbau produziert besonders die Leber große Mengen. Von der Leber gelangen die Ketonkörper über das Blut zu den anderen Geweben. Sie werden rasch aufgenommen, so dass normalerweise kaum Ketonkörper im Blut zu finden sind. Grundsätzlich lassen sich alle drei Ketonkörper mit chemischen Analysen im Harn bestimmen, in der Praxis wird aber meist nur der Harntstreifentest durchgeführt. Dies reicht auch im Allgemeinen aus. Es muss aber berücksichtigt werden, dass herkömmliche Streifentests das Beta-Hydroxybutyrat nicht erfassen. Es gibt aber einige Störungen, bei denen vor allem das Beta-Hydroxybutyrat erhöht ist, die anderen Ketonkörper weniger. Beispielsweise kann beim Start einer ketogenen Ernährung der Körper zwei unterschiedliche Arten von Ketonkörpern in etwa gleicher Menge herstellen (Azetoazetat und Beta-Hydroxybutyrat). Beide Formen dieser Ketonkörper werden zu Beginn sofort von den Muskeln etc. verbraucht oder über den Urin ausgeschieden. Im Laufe der Anpassung über ein paar Wochen verändert sich der Umgang des Körpers mit den zwei Formen. Die Muskeln hören auf, Beta-Hydroxybutyrat direkt aufzunehmen und nehmen stattdessen nur das Azetoazetat auf und reduzieren es zu Beta-Hydroxybutyrat. Dann geben sie es zurück in den Blutkreislauf. Deshalb kann nach ein paar Wochen der Anpassung nur noch eine Form der Ketone im Körper vorkommen. Diesem Sachverhalt muss bei der Analyse Rechnung getragen werden.

Hinsichtlich Drogentests über Urin gibt es solche, die nur nach einer Substanz suchen, als auch Tests, die gleich auf mehrere Substanzen testen. Üblicherweise werden Teststreifen für Opiate/Opioide (Heroin, Codein, Morphin etc.), Methadon, Speed (Amphetamin), Ecstasy Methamphetamin, Kokain, Cannabinoide, Barbiturate, Benzodiazepine und trizyklische Antidepressiva angeboten.

Bei Stuhluntersuchungen wird der menschliche Kot unter anderem auf mögliche Krankheitserreger und Blutbeimengungen untersucht. Letztere sind immer ein Anzeichen für eine Krankheit oder Verletzung und sollten ärztlich abgeklärt werden. Aber auch im gesunden Darm kommen zahlreiche physiologische und lebenswichtige Bakterien vor, insbesondere im Dickdarm. Gängige Nachweismethoden sind der Guajak-Test sowie immunologische Stuhlbluttests. Eine Stuhluntersuchung kann Hinweise auf verschiedene Erkrankungen liefern. Dazu zählen insbesondere Dickdarmkrebs, Darmpolypen oder Darmdivertikel, Durchfallerkrankungen oder Störungen des Gallenabflusses.

Insgesamt betrachtet wird die Aussage bestätigt, dass sich vieles über die Funktion und den Zustand einiger wichtiger Organsysteme des menschlichen Körpers mittels der Analyse von Exkrementen aussagen lässt.

Zum besseren Verständnis der Erfindung muss auch auf die vorhandenen gängigen Bauarten von Toiletten eingegangen werden. Weltweit gibt es sehr unterschiedliche Toilettentypen. Je nach Kultur und Einsatzgebiet können sich die Varianten stark unterscheiden. Die gängigsten Beispiele sind nachfolgend genannt.

Sogenannte Tiefspüler sind im Sitzen zu benutzende Toiletten, bei denen die Ausscheidungen in das Wasser eines Siphons fallen, der sich unter dem Gesäß des Benutzers befindet. Dadurch ist die Geruchsentwicklung gering, weil das Wasser den Kontakt der Exkremente mit der Raumluft vermindert. Ein Nachteil ist, dass das Wasser oft an das Gesäß hochspritzt. Diese Bauart ist die in westlichen Ländern am weitesten verbreitete Bauart. Dabei unterscheidet sich der Spülvorgang bei europäischen und nordamerikanischen Toiletten: während in Europa das beim Spülen einlaufende Wasser die Exkremente wegtransportiert, wird in Nordamerika in den oft mehrfach mäandernden Siphon ein Teil des Spülwassers als Wasserstrahl eingeleitet.

Eine Abwandlung dieses Spülsystems ist das Kaskaden-WC (auch Cascade-Spüler genannt), der Ablauf zum Siphon ist hierbei ganz hinten (wandseitig). Das Hochspritzen des Wassers wird durch eine Keramikzunge verhindert.

Sogenannte Flachspüler sind Sitz-Toiletten, bei denen sich unter dem Gesäß des Benutzers eine Art Stufe befindet, auf die die Ausscheidungen fallen. Der Ablauf zum Siphon ist beim Flachspüler vorne (zur Raummitte). Die Ausscheidungen verschwinden erst beim Spülen über den Siphon in das Abwassersystem. Der größte Nachteil dieser Bauart ist die starke Geruchsentwicklung, weswegen die Meisten öffentliche und private Toiletten seit den Neunziger-Jahren auf Tiefspüler umgerüstet wurden und werden.

Aktuell findet diesbezüglich ein weiterer Umbruch über die spülrandlosen WC's statt. Diese kommen ohne umlaufenden Rand aus, unter dem sich leicht Schmutz und Bakterien festsetzen. Das macht diese WC's hygienisch und pflegeleicht. Die Spülung arbeitet mit einem speziellen Spülverteiler, der das Wasser kraftvoll und möglichst spritzfrei nach rechts und links durch die WC-Schüssel leitet und in den Abfluss befördert. Am Markt gibt es zurzeit zwei unterschiedliche Typen von wasserrandlosen WC-Becken: Typ 1 ist spülrandlos mit einer kleinen Schürze an der Oberkante des Beckens. Bei Typ 2 ist der Spülrand von oben ins Becken komplett einsehbar, ohne kleine Schürze.

In Asien sind vor allem sog. Hocktoiletten bekannt. Bei einer Hocktoilette (manchmal auch Stehtoilette genannt) sitzt der Benutzer auf keiner Schüssel, sondern befindet sich in einer Hockstellung. Die Toilette kann dabei ein einfaches Loch oder eine Rinne im Boden sein, inzwischen gibt es aber auch größere, beckenähnliche Konstruktionen. Hocktoiletten sind vor allem in Asien, Südeuropa und islamischen Ländern verbreitet. Da kein Kontakt entsteht, werden sie oftmals als besonders hygienisch angesehen. Für Unerfahrene ist die Benutzung durchaus schwierig. Umgekehrt kann auch die Benutzung einer Sitz-Toilette ein Problem für Menschen darstellen, die daran nicht gewöhnt sind und den Kontakt zwischen Gesäß und WC-Brille als unhygienisch empfinden. Bemerkenswert ist bei der Hockstellung, dass - anders als bei Sitztoiletten - der Enddarm nicht abgeknickt wird.

Auch sind Vakuumtoiletten bekannt, welche in Flugzeugen, auf Schiffen, modernen Zügen sowie in der bemannten Raumfahrt eingesetzt werden. Toiletten im Weltraum sind wegen der fehlenden Schwerkraft nach einem staubsaugerähnlichen Prinzip konstruiert. Die Öffnung ist nur etwa handtellergroß, und die Benutzung muss trainiert werden.

Bei sog., Chemietoiletten' ist der Vorteil, dass das Abwasser weniger oft entsorgt werden muss. Nachteil sind die umweltschädlichen Chemikalien, welche zur chemischen Behandlung der Abwässer eingesetzt werden. Es ist aber auch bereits bekannt, die Abwässer biologisch zu behandeln. Dabei wandeln Mikroorganismen das Abwasser so um, dass der Wasseranteil wieder als Brauchwasser verwendet werden kann. Diese 'Biologischen Toiletten' ermöglichen lange Entleerungszyklen. Nachteil ist, dass das System "umkippen" kann, wenn beispielsweise die Toiletten-Muschel mit den falschen Chemikalien gereinigt wird.

Im Stand der Technik sind bereits unterschiedlichste Formen von "intelligenten" Toiletten beschrieben. Bei nahezu allen Lösungen ist die maßgebliche Technik entweder direkt in die Bauform der Toilette und/oder des Toilettensitzes integriert. Darüber hinaus wird Urin gesammelt, um ihn separat einer Analyse zuzuführen. So wurden allein von der Toto LTD bzw. Tochtergesellschaften zwischen den Jahren 1987 und 2005 über 25 Patente zu Analysemethoden in der Toilette offenbart, die eine bauliche Verbindung bedingen.

Aus der DE 60 12 64 48 T2 ist eine Urintestvorrichtung mit einem Biosensor bekannt, der auswechselbar innerhalb einer Urinsammelvorrichtung angebracht ist. Darüber hinaus weist die Urintestvorrichtung einen Signalprozessor auf. Die Messdaten von Patienten werden an ein Gesundheitsfürsorgezentrum übermittelt. Prävention oder Früherkennung werden nicht erwähnt. Der beschriebene Urinsammelbehälter ist unter hygienischen Gesichtspunkten problematisch.

In der DE 695 20 850 T2 wird eine Toilette beschrieben, welche über ein Urinsammelteil sowie ein Messteil zur optischen Untersuchung von Urin verfügt. Die Analyse des Urins findet bei diesem System in einer transparenten, rohrförmigen Flusszelle statt.

Die DE 10 2010 06 10 35 B4 beschreibt eine Toilette mit integrierter Messvorrichtung zum Erfassen physiologischer Daten eines Benutzers, wobei die Toilette eine im Innenvolumen des Toilettenbeckens vorgesehene Urinauffangvorrichtung samt Urinentnahmeleitung besitzt. Die Analyse findet in einer außerhalb des Toilettenbeckens vorgesehenen Küvette (Analyseeinheit) statt. Dort findet sich auch ein Farbindikatorver- und -entsorgungssystem, das mit einem Farbindikator-Streifenvorratsmagazin oder einer Farbindikatorrollenvorrichtung gekoppelt ist. Vornehmlich sollen Färbung und Trübung sowie Glukosegehalt der Urinprobe erfasst werden.

In der DE 34 02 488 C2 ist eine Toilette mit einem beweglichen Biosensor, einem Mikroprozessor und einer Anzeige zum Anzeigen der berechneten Ergebnisse des Biosensors beschrieben. Der Sensor nimmt Exkrement auf halber Höhe zwischen Toilettensitz und Toilettenbecken auf. Dazu ist ein beweglicher Arm vorgesehen.

Die DE 69117 229 T2 offenbart ein Toilettensystem, das ebenfalls mit einer Urinprobenentnahme und -analysefunktion versehen ist. Die Toilette weist eine schwenkbare Fördereinrichtung für Urin-Teststreifen auf. Streifen werden von der Fördereinrichtung in einen Probenhohlraum mit Urin getaucht, anschließend hochgezogen, optisch analysiert und danach in einem Abfallkasten entsorgt.

Mit der WO 2009 10 79 88 A2 ist eine Toilette bekannt, bei der Urin in real-time analysiert werden kann. Durch speziell angepasste infrarot ATR-Spektroskopie (Attenuated Total Reflectance) kann die Analyse in der dafür angepassten Toilette stattfinden. Dabei berechnet ein Algorithmus anhand der gewonnen Daten die Inhaltsstoffe des zuvor gesammelten Urins.

Die WO 2012 07 79 33 A2 beschreibt eine Urinsammeleinrichtung für eine am Toilettensitz montiertes Urinanalysesystem. Dabei wird der Urin in einem kleinen Becher gesammelt, der über ein mit Elektromotor angetriebenes Gestänge in den Toilettenraum geführt wird. Der Urin wird zur Analyse zurück in das sich im Toilettensitz befindliche Gerät gefahren.

Auch die WO 2012 10 57 48 A1 offenbart eine Lösung durch einen Toilettensitz. Dabei wird ein Urinteststreifen über eine ausgeklügelte Mechanik bestehend aus Eingabeschlitz, Eingabemodul und Trägersystem zwischen die Beine der auf dem Toilettensitz befindlichen Person transportiert. Im Anschluss wird der Testreifen nach Analyse über ein Entladungsmodul wieder nach draußen geführt. Ein Reinigungssystem sorgt für die Hygiene der Maschinerie.

Weitere Vorrichtungen sind aus US 5 625 911 A, US 4 466 445 A und DE 34 02 488 A1 bekannt. Vorrichtungen, bei denen dem Mobilitätsgedanken in der Anwendung teilweise Rechnung getragen wurde gibt es sehr wenige. Die WO 2009/35599 A1 beschreibt ein an verschiedenen Toilettentypen anbringbares Gerät, welches Urin sammelt und dann einer Auswertung zuführt. Es gibt dabei einen Urinempfänger und ein Urinsammelgefäß., was wiederum Probleme bei der Hygiene mit sich bringt. Die Ausführungen sind teilweise groß und flächig in der Toilette angebracht. Bei dieser Vorrichtung wird kein Indikator genutzt. Es ist für den einfachen, diskreten Gebrauch durch einen Nutzer unzweckmäßig.

Die US 2005/0261605 A1 beschreibt eine Vorrichtung, die an unterschiedlichen Toilettentypen angebracht werden kann. Sie sammelt den Urin zunächst in einer Art Becher und wertet ihn anschließend aus. Zur genauen Kalibration oder Probename bei unterschiedlichen Physiognomien wird nichts gesagt. Es bleiben Zweifel, ob es überhaupt möglich ist, während eines normalen Toilettengangs den seitlich angebrachten Becher zu treffen, ohne dabei unhygienische Spritzer zu erzeugen.

Die zuvor beschriebenen Limitationen und Probleme bleiben daher auch in dieser Ausführung bestehen. Die Analyse erfolgt wiederum nicht durch Indikatoren sondern durch eine optische Auswertung in der Kammer unterstützt durch diverse Methoden einer Beleuchtung.

Für eine erfolgreiche Anwendung einer regelmäßigen automatisierten Untersuchung von Exkrementen darf der "normale" Toilettengang nicht wesentlich beeinträchtigt werden. Darüber hinaus muss ein gewisser Hygienestandard gewahrt werden. Sofern die Toilettennutzer selbst mit den Ausscheidungen in Kontakt kommen, sie abfüllen oder auf andere Art beseitigen müssen, ist eine enorm hohe Barriere gegeben. Kaum eine Person, die nicht dazu verpflichtet ist, wird sich regelmäßig, freiwillig solch einem Unterfangen unterziehen. Allerdings ist mit den aufwendigen Anbringungs- und Benutzungsherausforderungen bisheriger Umsetzungsansätze sowie den notwendigen baulichen Änderung beim Gros der Lösungen eine der wesentlichen Hürden für den breiten Einsatz gegeben. Insbesondere scheuen viele Benutzer den baulichen Aufwand, ihre alte Toilette herauszubauen und durch eine neue, "intelligente" Toilette zu ersetzen.

Darüber hinaus bietet keine bekannte Umsetzung den Brückenschlag zu heutigen oder zukünftigen Verarbeitungs- und Kommunikationsmedien beispielsweise durch mobile Endgeräte. Die Visualisierung, Aufbereitung und intelligente Weiterverarbeitung in Form von Apps oder moderneren Softwareanwendungen ist nahezu nicht vorhanden.

Davon ausgehend liegt der Erfindung die Aufgabe zugrunde, die eingangs genannten Vorrichtung und Verfahren zur Bestimmung von Gesundheitsdaten durch Analyse von menschlichen Exkrementen in einer Toilette durch Messung von Urin- und/oder Stuhlwerten so auszugestalten und weiterzuentwickeln, dass bei vertretbarem konstruktiven Aufwand und überschaubaren Kosten ein möglichst universeller Einsatz und somit eine weite Verbreitung auch bei unterschiedlichsten Bauarten von Toiletten erreicht werden kann. Weiterhin ist auch eine mobile Verwendung der Vorrichtung erwünscht.

Vorrichtungsmäßig erfolgt die Lösung der Aufgabe gemäß Anspruch 1 durch ein Messsystem, bei dem für jede Messung ein Indikator bzw. Sensor in eine Messposition bringbar ist, in der ein ausreichender Kontakt mit dem zu untersuchenden Urin oder Stuhl erfolgt, wobei der Indikator bzw. Sensor als eigenständige Einheit ausgebildet ist/sind.

Die Aufgabe wird bei einem Verfahren nach Anspruch 11 dadurch gelöst, dass eine Mehrzahl zuvor bestimmter Werte gemessen und anschließend, bevorzugt automatisiert, weiterverarbeitet und ggf. weitergeleitet

Aspekte, Vorrichtungen, Verfahren, Ausführungsformen sowie Ausführungsvarianten, die hier beschrieben sind und nicht unter den Wortlaut der Patentansprüche fallen, sind nicht Teil der Erfindung.

In den prioritiätsbegründenden Patentanmeldungen DE 10 2015 112 678.8 und DE 10 2016 107 486.1 wurden bereits eine Vorrichtung und ein Verfahren beschrieben, mit der sich die mobile Analyse von Exkrementen in der Toilette durchführen lässt.

Zum besseren Verständnis sind nachfolgend wesentliche Aspekte und Bestandteile von Ausführungsformen sowie Ausführungsvarianten näher erläutert:

### 1. Analyseeinrichtung

Mit "Analyseeinrichtung" ist die Gesamtheit der in und an der Toilette angebrachten Systembestandteile gemeint. Vornehmlich werden diese in einem physischen Ort (dem Gerätegehäuse) zusammengefasst sein. Es kann aber auch der Fall sein, dass beispielsweise die optoelektrische Einheit und der Sensorarm in zwei unterschiedlichen Gerätehüllen verortet sind. Der relative Abstand zueinander kann somit ebenfalls variieren. Die Kommunikation bzw. Abstimmung der Systemteile miteinander würde über Funk erfolgen.

Die Anbringung der Analyseeinrichtung an oder in der Toilette kann durch ein/en Adhäsiv, Kleber, mittels einer Haken- oder Bogenvorrichtung ähnlich wie sie bei Toilettensteinen oder Toilettenreinigungsvorrichtungen genutzt werden, erfolgen.

Darüber hinaus ist auch vorstellbar, die Messvorrichtung durch Saughalter, Magnetismus (an Edelstahltoiletten direkt oder durch Gegenmagnet an normalen Toiletten), Klebevorrichtungen oder andere gängige Anbringungsmethoden an einer geeigneten Stelle in oder außerhalb der Toilette anzubringen. Der Ort der optimalen Anbringung variiert nach Bauform der Toilette, wird sich in einem gängigen Tiefspüler vorzugsweise direkt am oder unterhalb des Spülrandes befinden, um ggf. etwas Platz einzusparen und um die Distanz zum Messbereich in der Toilette zu verringern. Bei moderneren, randlosen Toiletten wird das Gerät soweit oberhalb der Spülfuge bzw. des Wasserstroms positioniert sein, dass kein fortlaufender Kontakt mit Spülwasser entsteht. Die Anbringungsmethoden sind prinzipiell identisch. Auch ist denkbar das Gehäuse materialseitig flexibel auszugestalten, so dass es sich an die Toilettenform, optimal anpasst. Im einfacheren Fall liese sich dieser Effekt auch durch einen modularen Aufbau des Gehäuses darstellen, bei dem einzelne Bereiche über Scharniere oder bewegliche Elemente miteinander verbunden sind.

### 2. Indikator/Sensor

Als Indikator/Sensor können unterschiedliche Vorrichtungen und Verfahren dienen.

Das gängigste Verfahren wird über Teststreifen, Testpapier oder im weitesten Sinne über Einmaltestträger funktionieren. Dabei wird ein Testträger so präpariert, dass er Analysen durchführen kann. Durch die Veränderung des Indikators ergibt sich die Möglichkeit der Analyse des Urins. Der Indikator wird entweder an Ort und Stelle der Kontaktnahme ausgewertet (meist durch optoelektrische oder sonografische Einheit) oder zurück an oder in das Gerät zur weiteren Analyse geführt. Üblicherweise braucht sich der Testträger nach einmaligem Gebrauch auf und wird anschließend entsorgt.

Darüber hinaus gibt es Mehrfachindikatoren, beispielsweise Nano-Biosensoren, die sich nicht nach einmaligem Gebrauch abnutzen und somit mehrfach einsetzbar sind. Die Messung erfolgt im Wesentlichen über elektrische Signale.

Orte zur Präsentation des Indikators können von der Stelle des Körperverlassens bis zum Abflussrohr sein. Insbesondere wird es in gängigen Versionen entweder im Siphon, knapp darüber und vor allem im unteren, vorderen Bereich der sein.

### 3. Kalibrieren & Justieren

Unter Kalibrieren versteht man das Feststellen und Dokumentieren der Abweichung der Anzeige eines Messgerätes oder einer Steuereinheit vom richtigen Wert der Messgröße. Beim Justieren wird das Messgerät so eingestellt bzw. abgeglichen, dass die Messabweichungen vom Sollwert möglichst klein werden und innerhalb der Gerätespezifikationen liegen. Im vorliegenden Fall sind mehrere Situationen bekannt, bei denen eine der beiden Maßnahmen notwendig ist. Oft hängt das Justieren aber eng mit Kalibrieren zusammen.

Der erste Fall stellt sich bei der Anbringung des Gerätes an der Toilette dar. Je nach Bauform der Toilette bietet sich ein großes oder kleines Anbringungsfenster am linken oder rechten Rand der Toilette. Nur innerhalb dieses Fensters kann der Sensorarm zuverlässig und genau den optimalen Ort zur Probenahme erreichen. Dieser Ort kann mit Kenntnis des Toilettenmodels, der grundsätzlichen Bauform oder unter Zuhilfenahme der optischen Messinstrumente am Gerät bestimmt werden. Das Feedback an den Nutzer, dass der optimale Anbringungsort erreicht ist, kann beispielsweise optisch oder akustisch erfolgen.

Bedingt durch unterschiedliche Bauformen von Toiletten muss auch der jeweils optimale Ort zur Probenahme bestimmt werden. Dies passiert ebenfalls durch Kenntnis des Toilettenmodels, der grundsätzlichen Bauform oder unter Zuhilfenahme der optischen Messinstrumente. Zusätzlich wird dabei auch die Ausfahrlänge des Sensorarms bestimmt, sowie der Winkel, in dem der Indikator optimal an der Toilettenwand präsentiert wird. Hier kann unterstützend der Sensorarm ausfahren und neben der optischen Erkennung auch eine drucksensorische Erkennung ermöglichen, sobald der Arm beispielsweise die Toilettenwand berührt. Getroffene Einstellungen werden gespeichert, um eine wiederholte Anbringung beispielweise bei Abnahmen des Gerätes zur Reinigung der Toilette zu erleichtern. Nachkalibrierungen bei schlechten Probenahmen/Ergebnissen, ab einer gewissen Einsatzzeit oder -zahl sind ebenfalls möglich.

### 4. Auslösung der Messung

Die Auslösung der Messung kann durch folgende Mechanismen erfolgen, die mitunter auch kombiniert werden können:
- Auslösung durch Betätigung eines Hebels oder Tasters, der entweder direkt mit den System verbunden ist oder alternativ durch Kabel/Funk.
- Lichtschranke/optische Einheit, die analysiert, wenn Urin /Kot/Indikator in den Analysebereich eintritt.
- Auslösung durch ein mobiles Endgerät.
- Drucksensor bei Gewicht auf Toilettenbestandteil (beispielsweise Körpergewicht auf Toilettenbrille) oder Fuß-Bodenkontakt bei Urinal.
- Akustisches Signal: Beispielsweise ein Ruf, eine Melodie oder ein Pfiff.

### 5. Sensorarm

Über den Sensorarm wird der Indikator/Sensor an die jeweils geeignetste Stelle innerhalb der Toilettenschüssel präsentiert. Je nach Bauform wird dies im Gros der Fälle an einem der tiefsten Punkte knapp oberhalb des Siphonwassers sein.

Die Präsentation kann über verschiedene Prinzipien erfolgen. Im weitesten Sinne ist es eine Vorrichtung, die die Distanz zwischen Exkrement und Indikator/Analyseeinrichtung überbrückt. Darüber hinaus wird die Vorrichtung mit einer Halterung oder Aufnahmeeinheit für den Indikator/Sensor versehen sein.

### 6. Messeinheiten

Die Messeinheiten können je nach zu messendem Medium und/oder genutztem Indikator auf unterschiedliche Weise funktionieren.

### 6.1. Optoelektrische Auslesung eines Indikators/Sensors

Dabei wird der Indikator/Sensor durch eine optoelektrische oder auch optische (Auslese-) Einheit ausgelesen. Unter diesen Begriffen werden im weitesten Sinne alle Produkte und Verfahren verstanden, die sowohl die Aufnahme von Bildern als auch die Umwandlung von elektronisch erzeugten Daten und Energien in Lichtemission ermöglichen und umgekehrt. Solche sind beispielsweise klassische Fotoaufnahmen (bevorzugt digital), bildgebende Verfahren aus der Medizin, Konfokaltechnik, Laserscanning, Thermografie, Terahertzstrahlung etc..

Es ist sehr wahrscheinlich, dass Nutzer aus Gründen der Privatsphäre keine großformatigen Fotoaufnahmen des Toiletteninneren sehen oder gespeichert haben wollen. Daher werden die optoelektrischen Aufnahmen wahrscheinlich sehr fokussiert auf den Indikator oder in einem angepassten Format erfolgen (beispielsweise schematisiert).

Eine sinnvolle Alternative bietet die Auswertung direkt am Sensorarm. Dabei wird eine kleine Kamera direkt am vorderen Teil des Sensorarms oder der Führungshülse für den Sensorarm angebracht und kann somit direkt eine Auswertung bei Kontaktaufnahme der Indikatoreinheit mit Exkrement vornehmen. Unterstützend kann hierbei die Anbringung von Lichtquellen, bspw. LED, ebenfalls am Ende des Sensorarms sein. Dadurch wird eine optimale Ausleuchtung der Indikatoreinheit gewährleistet. Die Kamera kann einzelne oder Serien von Bilder machen, um den Verlauf ebenfalls zu dokumentieren.

### 6.2. Sonografische Auslesung eines Indikators/Sensors

Unter dem Begriff Auslesung über eine sonografische Einheit sind im weitesten Sinne alle Produkte und Verfahren zusammengefasst, die es erlauben, Werte über Schallwellentechnologie zu bestimmen. Sonografie beruht dabei zumeist auf dem Echoprinzip. Ein gerichteter (Ultra-)Schall wird ausgesandt und von den aufeinanderfolgenden Schichten des beschallten Objektes mehr oder weniger stark reflektiert (beispielsweise luftgekoppelte Ultraschallprüftechnik). Aus der Laufzeit des reflektierten Signals kann die Schichtstruktur des Objekts rekonstruiert werden. Vorteile gegenüber der optoelektrischen Technik liegen insbesondere dann vor, wenn beispielsweise optische Methoden wegen schlechter Ausleuchtung der Oberfläche nicht zum Einsatz gebracht werden können.

### 6.3. Elektrische Messung eines Indikators/Sensors

Vor allem bei den Nano-Biosensoren wird die Messung über elektrische Ströme, d.h. auch deren Stärke oder Länge stattfinden. Beispielsweise. bei Kontakt eines Rezeptors mit einem Effektor wird ein elektrischer Impuls erzeugt, der anschließend gemessen und ausgewertet wird.

### 6.4. Optische Volumen- und Gewichtsmessung der Exkremente

Mit Unterstützung einer oder mehrerer optischer (beispielsweise 3D-Scanner) oder sonografischer Einheiten kann das System mit einer sehr geringen Toleranzquote die Höhe, Breite und Länge eines Körpers messen. Die Genauigkeit der Messung könnte durch weitere Kameras bis auf 99 Prozent erhöht werden. Neben der Volumenmessung kann auch das Gewicht ermittelt werden. Die gesammelten Daten können beispielsweise auf mobilen Endgeräten verwaltet und ggf. auch mit anderen geteilt werden. Bevorzugt wir dies in Flachspültoiletten erfolgen kann aber auch in weiteren Toilettentypen umsetzbar sein.

### 6.5. Verlaufsmessung

Die optoelektrische Auswerte- und Analysemethode, mit der die Urinprobe untersucht wird, bietet die Möglichkeit den zeitlichen Verlauf der Indikatorreaktion zu verfolgen. Dies erfolgt bspw. durch die Aufnahme mehrerer Bilder pro Sekunde. Diese zusätzliche, zeitliche Information lässt unter Umständen Rückschlüsse auf Quantitäten aber auch Qualitäten von Inhaltsstoffen in Exkrementen zu. Bei unterschiedlichen Konzentrationen können manche Indikatoren im zeitlichen Verlauf unterschiedlich reagieren.

### 7. Magazin/Indikatorlager

Bei den meisten Ausführungsformen wird ein Indikator genutzt, der sich aufbraucht und somit wieder und wieder "nachgeladen" werden muss. Dabei können je nach Kundenwunsch unterschiedliche Tests durchgeführt werden. So kann eine Vorrichtung mehrere Magazinfächer besitzen, in die unterschiedliche Test "hineingeladen" werden können, beispielsweise ein Magazin mit Tests von Standard-Urinwerten, eines mit Schwangerschaftstests und eines mit Ovulationstests. Beim Toilettengang wählt der Kunde, meist durch Betätigen eines Knopfes an der Kontrolleinheit, welcher Test nun vollzogen werden soll. Bei der Messung von Urin können Urinteststreifen genutzt werden, wie sie überall in Apotheken zu bekommen sind. Diese Streifen können, sofern die spezielle Gerätevariante das zulässt, in definierten Stückmengen in das System geladen werden. Das Gerät entnimmt bei jedem Test ein Streifen aus dem Magazin (passiert durch beispielsweise durch schlichte Schwerkraft, d.h. fällt in den Auswurf, durch Unterdruck, eine Feder, einen Schieber etc.), und präsentiert ihn dem Nutzer auf den beschriebenen Wegen. Denkbar wär hier auch ein revolvierendes System, in dem sich die Streifen in Kammern einsortieren. Allerdings werden, schon allein aus Gründen des geringen Platzes, meist kleinere Testplättchen in einem eigens dafür angepassten Magazin für den Einsatz vorgesehen werden.

So kann auf der Innenseite der Toilette auch ein Entsorgungsfach ("Sammeleinheit") angeordnet sein, in das die benutzten Indikatoreinheiten entsorgt werden. Falls es sich bspw. um biologisch abbaubares Material handelt, ist eine Entsorgung über die Spülung ebenfalls möglich. Auch sind ein flüssig vorgehaltener Indikator oder Indikator oder Indikator in Rollenform zum Abreißen bzw. Trennen einsetzbar.

### 8. Auswerteinrichtung

Mit "Auswerteinrichtung" ist quasi das "Gehirn" des Systems gemeint. Die Auswerteinrichtung nimmt die durch die Analyse generierten Daten auf und vergleicht sie beispielsweise mit Referenzdaten aus einer Datenbank. In einer gängigen Variante ist dies eine Art Minicomputer mit Leistungsmerkmalen ähnlich denen eines Smartphones. Bei einer extrem schlanken Ausgestaltung der Analyseeinrichtung in oder an der Toilette, kann Rechenleistung auch nach "aussen" gegeben werden und es werden lediglich die "Rohdaten" der Analyse an ein Endgerät gegeben. Eine Anwendung, die auf dem Endgerät läuft, bereitet alle relevanten Daten auf und visualisiert diese. Geräte zur Visualisierung sind insbesondere mobile Endgeräte.

Als mobile Endgeräte oder "mobile Computing" verstehen sich im weitesten Sinne Produkte oder Verfahren, die elektronische Datenverarbeitung auf tragbaren Computern erlauben. Dies sind beispielsweise Smartphones, Laptops, Tablets oder Wearables wie Brillen, Fitness- Armbänder, Uhren, "smarte" Kleidung und Minicomputer im menschlichen Körper, also Gegenstände, die aus unserem heutigen und zukünftigen Leben nicht mehr wegzudenken sind.

### 9. Kommunikationseinrichtung

Die Kommunikationseinrichtung wird vorzugsweise ein Bestandteil der Analyseeinrichtung sein. Von dort erfolgt die Weitergabe der Daten an Endgeräte zur Visualisierung entweder durch kabelgebundene als auch kabellose Übertragungsstandards, wie sie üblicherweise im häuslichen Umfeld genutzt werden. Beispielsweise Bluetooth, W-lan, Low-Energy Übertragungsstandards etc.. Denkbar wären auch akustische- oder Lichtsignale, sofern sie von der Kommunikationseinrichtung aufgenommen und verarbeitet werden können.

### 10. Visualisierung

Neben der Visualisierung über Software-Anwendungen auf mobilen Endgeräten wäre auch ein simpler Monitor möglich, der die Werte und Daten direkt visualisiert und ggf. an ein geeignetes Speichermedium weitersendet. Der Monitor kann entweder direkt an der Analyseeinrichtung angebracht sein oder getrennt davon an einer anderen, besser sichtbaren Stelle an, beispielsweise auf Augenhöhe an einer seitlich zur Toilette gelegenen Wand. Übertragen wird die Informationen durch die zuvor beschriebenen Kommunikationsstandards.

Speziell für ältere Menschen kann man sich auch vorstellen, dass die Werte auf einer Art Beleg von einem Gerät ausgedruckt werden (beispielsweise auf Thermopapier). Kritische oder beachtenswerte Werte können hierbei direkt hervorgehoben werden. Mit dieser Frühindikation können Nutzer entsprechend zur ärztlichen Vorsorge gehen und auffällige Werte genauer interpretieren lassen.

### 11. Energieversorgung/Energiespeicherung

Die notwendige Energie zum Betrieb des Systems wird nicht durch direkten Anschluss an eine herkömmliche Steckdose bezogen. Innerhalb oder in unmittelbarer Nähe zum Toilettenspülsystem mit Energie zu arbeiten, die potentiell einen Menschen umbringen könnte, erscheint nicht ratsam. Im Umkehrschluss bedeutet dies, dass das System seine Energie anderweitig beziehen muss. Zuvorderst sei hier der Betrieb über Batterien bzw. Akkus genannt. Als Batterie können hier alle gängigen Formate genutzt werden, die sich von der Größe her noch im Geräte unterbringen lassen. Beispielsweise AA, AAA, Knopfzellen etc. Die Batterie können entweder wieder aufladbar oder Einmalartikel sein. Als weiter Energieversorgungsform kommt insbesondere auch Solarbetrieb (meist gekoppelt mit Batterie) infrage. Ähnlich, wie man es bei Taschenrechnern kennt, versorgt eine Zelleneinheit, die entweder direkt am System oder extern (dann mit Kabel daran angeschlossen) angebracht ist, das ganze System mit der notwendigen Energie.

### 12. Hygiene

Hygiene bezeichnet in vorliegenden Fall die Maßnahmen zur Reinigung, zur Desinfektion und zur Vorbeugung von Verschmutzungen insbesondere der Vorrichtung aber auch der Toilette
Dazu gibt es mehrere Ansätze die größtenteils auch miteinander kombiniert werden:
-. Grundsätzlich minimalistischer Aufbau des Gerätes auf der Innenseite der Toilette. Dadurch bietet sich weniger Angriffsfläche für Verschmutzung
-. Sehr kurze Verweildauer des Sensorarms zur Probenahme. Kombiniert mit einem sehr dünnen Sensorarm reduziert sich die Wahrscheinlichkeit einer Anhaftung von Urin und Keimen erheblich.
- Vibration des Sensorarms sowohl zum Entsorgen des Testblättchens als auch zum Reinigen des Sensorarms.
- Das Gehäuse erhält eine Schutzhülle, die regelmäßig erneuert werden kann. Die "alte" Hülle wird über den normalen Restmüll entsorgt.
- Modularer Aufbau. Dadurch können einzelne Teile separat gereinigt oder ggf. ersetzt werden,
- Vorteilhafte Materialbeschichtungen. Dies erschwert grundsätzlich die Anhaftung unerwünschter Stoffe.
- UV-Licht Quellen an der Vorrichtung. So genanntes «fernes» UV-Licht ist in der Lage, 99,9 Prozent aller Bakterien und Pathogene auf einer Oberfläche zu eliminieren.

Die erfindungsgemäße Lösung sieht mehrere Varianten zur Analyse vor. Allen ist aber gemein, dass sie die nachträgliche Nutzung durch einfache Nachrüstung von Bestandstoiletten erlauben. Der "Plug 'n' Play Charakter" spielt eine sehr wichtige Rolle zur großflächigen Etablierung der erfindungsgemäßen Idee und für die Akzeptanz einer regelmäßigen Benutzung.

Die Erfindung umfasst eine Vielzahl unterschiedlicher Messsysteme: vom einfachen, manuell zu betätigenden Messgerät bis hin zu elektrisch betriebenen Einrichtungen, die den Messvorgang automatisiert durchführen. Darüber hinaus kann bei allen Systemen eine Auswertung stattfinden. In der einfachsten Form als schlichte Referenzskala, in den meisten Fällen aber computerunterstützt. Häufig kommunizieren die Systeme ihre Daten an Auswerteinrichtungen. Diese sind entweder direkt an das System angeschlossen und ausschließlich für diesen Zweck konzipiert oder sie bieten (meist auch hauptsächlich) andere Funktionen und das System nutzt lediglich die Rechen- und Visualisierungskapazität dieser (beim Benutzer bereits vorhandenen) mobilen Endgeräte.

Bezüglich Nutzung der Vorrichtung ist es sehr wichtig, den exakten Zeitpunkt zur Messung zu kennen. Besonders bei älteren Menschen bedeutet ein absetzen auf der Toilettenbrille nicht unbedingt, dass sofort Urin kommt. Aus unterschiedlichen Gründen kann dies bei den verschiedenen Nutzern unterschiedlich lange dauern. In einer bevorzugten Ausführung der Vorrichtung können daher mehrere der zuvor genannten optoelektrischen oder sonografischen Methoden angewandt und teilweise miteinander kombiniert werden, um den optimalen Messzeitpunkt zu bestimmen.

Eine weitere Variante zur Bestimmung des Messzeitpunktes ist die Verwendung eines Wärmesensors an der Vorrichtung. Bei entsprechenden Temperaturunterschieden oder bei Erreichen eines genauen Zielwerts (beispielsweise Temperatur von Urin) wird die Messung auslöst. Eine weitere Methode ist über die Ermittlung von Geräuschen (beispielsweise Plätschern), die für das Ausscheiden charakteristisch sind. Ebenfalls möglich ist die Bestimmung des Zeitpunktes durch optische Messtechnik (beispielsweise Reflexion). Eine Lichtschranke und/oder eine Laser sind auf Punkte in der Toilette ausgerichtet an denen Exkrement ankommen muss. Durch die Veränderung der optischen Eigenschaften der Oberfläche der Messpunkte durch Exkrement, wird die Messung ausgelöst.

Eine weitere Lehre der Erfindung sieht vor, mit optoelektrischen Auswerte- und Analysemethoden den zeitlichen Verlauf der Indikatorreaktion zu verfolgen. Durch die Aufnahme mehrerer Bilder pro Sekunde, vom Zeitpunkt des ersten Probekontaktes bis zum Ende der Reaktion mit dem Indikator/Sensor, lassen sich unter Umständen Rückschlüsse auf Quantitäten aber auch Qualitäten von Inhaltsstoffen in Exkrementen zu. Bei unterschiedlichen Konzentrationen können manche Indikatoren im zeitlichen Verlauf unterschiedlich reagieren.

Um eine eindeutige Benutzerzuordnung und Datensicherheit zu gewährleisten, kann an dem Gerät in einer weiteren Ausführungsform auch ein Fingerabdrucksensor oder eine Spracherkennung vorgesehen sein.

In einer bevorzugten Ausführungsform ist der Sensorarm aus einem vorzugsweise ausrollbaren Element gebildet, welches in die Messposition bewegt werden kann. Der Sensorarm hat in weiterer Ausgestaltung der Erfindung eine selbstversteifende Eigenschaft.
Dabei rollt sich der Sensorarm aus und formt einen "Arm". Nach erfolgter Messung wird das Element aufgerollt und der "Arm" wird wieder verstaut. Das dafür verwendete Material besitzt eine inhärente Spannung, die durch mechanische Verformung wie beispielsweise durch Führungen, Umlenkrollen oder -lippen entweder zum Versteifen des "Arms" führt, oder den "Arm" wieder flexibel und aufrollbar macht. Der versteifte Arm kann rohrförmig geschlossen oder halboffen ausgeführt sein. Am Ende dieses Elementes sind - wie auch in den anderen bevorzugten Ausführungsformen - Halterungen zur Aufnahme, zum Transport und zur Entsorgung des Indikators angebracht. Diese Version ist platzsparend, und weniger anfällig als andere mechanische Umsetzungen. Die Länge des Arms kann dadurch sehr variabel gestaltet werden, da der Platzbedarf des aufgerollten Elements in der mobilen Vorrichtung praktisch unabhängig von der "Armlänge" ist.

Ein ausrollbarer selbstversteifender "Arm" ist für sich bereits bekannt und beispielsweise in der US 6 602 574 B1 beschrieben.

Der selbstversteifende Sensorarm ist zwischen einer aufgewickelten und einer ausgezogenen Form umkehrbar konfigurierbar. Beim Ausrollen versteift sich das Element und stellt den "Sensorarm" dar. Die Versteifung kann durch eine entsprechende Materialwahl, die beispielsweise Zug- und/oder Druckkräfte um die ausfahrbare Achse aufweist, erfolgen. Zusätzlich kann sich das Material beispielsweise zu einer Kreisform um die ausfahrbare Achse verformen. Selbiger Effekt kann aber auch durch eine Umlenklippe generiert werden. Dabei wird das Element, das ähnlich einem ausziehbaren Metermaß aufgerollt ist, beim Verlassen des Gehäuses über eine Vorrichtung so in die Ausrollung gezwungen, dass es beispielsweise in eine Form mit rundem oder eckigem Querschnitt gebracht wird und dadurch unmittelbar eine Versteifung mit sich bringt. Das bedeutet, die Geometrie des Elements erzeugt die notwendige Stabilität für die Analyse. Am Ende des Elementes sind, wie auch in den anderen Ausführungsformen, Halterungen zur Aufnahme, zum Transport und zur Entsorgung des eigentlichen Indikators angebracht.

In einer weiteren bevorzugten Ausführungsform ist das angetriebene Ende des Sensorarms an der Außenseite der Toilette angebracht und rollt sich quasi über den Haltebügel (zwischen Toilettenschüssel und -sitz) ins Toiletteninnere aus. Vorzugsweise ist im Inneren des Haltebügels eine dafür vorgesehene Führung angebracht. Das könnte beispielsweise eine Umhüllung wie ein Schlauch oder eine Plastikfassung sein, aber auch einfache Klammern oder Umlenkobjekte sind möglich. Auch kann für diese Variante eine Form des Bowdenzugs genutzt werden. Dieser bewegt sich per se in einer Ummantelung und kann flexibel lang ausgestaltet sein.

Technologisch kann die optische Messtechnik in einer weiteren bevorzugten Ausführungsform auch durch (Zeilen-) Kamera(s) in Kombination mit (optischen) Referenzmustern (wie Linien, Gitter, etc.) durchgeführt werden. Durch eine entsprechende räumlichen Auflösung und Zuordnung sowie höherer Geschwindigkeit können hochwertige dreidimensionale Aufnahmen von Bereichen innerhalb der Toilette gemacht werden. Neben der Auslösung der Messung kann dies vor allem auch zur Volumen- und Massemessung von Exkrement genutzt werden. Bei Urin bedeutet dies unter Umständen sogar zum Zeitpunkt vom Verlassen des Körpers bis zum Kontakt mit der Toilette. Über Algorithmen lässt sich mit den gewonnen Daten das Volumen, die Masse sowie der Volumen- und Massenstrom berechnen.

Die Kalibration und Justage der Vorrichtung sowie des Sensorarms können in einer weiteren bevorzugten Ausführungsform unter Zuhilfenahme unterschiedlicher Informationsquellen vollzogen werden. Neben Daten zuvor beschriebener Sensoren, die Standorte und relative Abstände zu Zielpositionen bestimmen, werden auch Informationen durch Kenntnis des Toilettenmodels oder Abgleich der grundsätzlichen Bauform genutzt. Das Feedback an den Nutzer kann optisch oder akustisch erfolgen.

Ähnlich wird in einer weiteren bevorzugten Ausführungsform der jeweils optimale Ort zur Probenahme bestimmt. Zusätzlich wird dabei die Ausfahrlänge des Sensorarms sowie der Winkel, in dem sich der Indikator optimal an der Toilettenwand präsentiert, bestimmt. Neben Nutzung der zuvor beschriebenen Informationsquellen kann der Sensorarm zu diesem Zweck ausfahren und drucksensorische Erkennung ermöglichen. Dies passiert wenn der Arm beispielsweise die Toilettenwand berührt. Getroffene Einstellungen werden bei allen Kalibrationen und Justagen gespeichert, um eine wiederholte Anbringung beispielweise bei Abnahmen des Gerätes zur Reinigung der Toilette zu erleichtern. Nachkalibrierungen bei schlechten Probenahmen/Ergebnissen, ab einer gewissen Einsatzzeit oder -zahl sind ebenfalls möglich.

Die Vorrichtung zur Stuhlanalyse gleicht der zur Urinanalyse. Beide Untersuchungen können durch eine Vorrichtung (Kombigerät) abgedeckt werden. Es ist allerdings auch möglich, nur eine, explizite, Vorrichtung dafür vorzusehen. Vorteil wäre, dass dabei auf den Sensorarm verzichtet werden könnte.

Als Indikator für die Stuhlanalyse wird speziell präpariertes Toiletten- oder Hygienepapier benutzt. Das Papier hat die Eigenschaft, nach Kontakt mit Stuhl und den darin enthaltenen Bestandteilen, vor allem denen, die nicht in gesundem Stuhl vorkommen sollten, die Oberflächenstruktur und insbesondere die Farbe zu verändern. Durch die optoelektrische oder sonografische Einheit kann im Anschluss diese Veränderung detektiert werden.

Dazu kann es notwendig sein (vor allem bei Kombigeräten), dass sich die Messeinheiten für diesen Anlass neu kalibrieren. Beispielsweise. kann sich der Focus einer Kamera bei einer Tiefspültoilette stärker zum Siphon hin verändern.

In einer weiteren bevorzugten Ausführungsform kommt beispielsweise eine Analyse zum Einsatz, bei der sich der Indikator/die Sensorik, im Gegensatz zu den zuvor beschriebenen Lösungen, nicht aufbraucht. Zu diesem Zweck wird der "Sensorarm" anstelle eines Einmalindikators mit einem mehrfach einsetzbaren Sensor versehen. Für die zugrundeliegende Erfindung gesprochen, wird es sich so verhalten, dass entweder ein universeller "Sensorkopf" alle vorstellbaren Analysen ausführen kann, oder, dass die Köpfe je nach Messwunsch ausgetauscht werden können. Bevorzugt wäre hier ebenfalls eine Art Magazinvariante, bei der je nach Messabsicht ein anderer "Messkopf" am Ende des Sensorarms angebracht wird. Unter "Messkopf" kann man hier verschiedene technische Umsetzungen verstehen, denen aber allen gemein ist, dass sie die Messung eines oder mehrere unterschiedlicher Stoffe erlauben. Umsetzungsansätze können beispielsweise zwei Antennen wie bei Kapazitiv-Füllstandsensoren für Flüssigkeiten sein, oder lediglich ein kleines Blättchen mit unterschiedlichen Nanorezeptoren oder auch eine Art "Hütchen", zum Anklicken auf das Ende des Sensorarms. Die Informationen werden durch den Sensor aufgenommen und elektronisch weitergegeben. Entweder zu einem Minicomputer in der Analyseeinrichtung oder sogar direkt an ein mobiles Endgerät.

Als besondere Form der Mehrfachindikation wird eine speziell behandelt Fläche (beispielsweise mit Nano-Biosensoren) benutzt, die sich in der Toilette, vorzugsweise dort, wo sie mit Urin überflossen wird, anbringen lässt. Nach Kontakt mit Urin verändert sich die Fläche durch Farb-und/oder Strukturänderung derart, dass sie durch die nachfolgend beschriebenen Messverfahren ausgelesen werden kann. Vorteil dieser Variante ist, dass komplett auf den Sensorarm verzichtet werden kann.

In einer anderen bevorzugten Ausführungsform wird die Indikatoreinheit in einer flüssigen Form genutzt. Dazu wird Indikatorenzym von der Haupteinheit über ein Schlauchsystem zum Ende des Sensorarms transportiert. Dort verteilt sich das Enzym auf ein Gitternetz. Durch speziell dimensionierte Gitterflächen und Materialien verteilt sich und haftet das Enzym auf der gesamten Fläche. Dieses Gitternetz wird mittels des Sensorarms mit Exkrement in Kontakt gebracht und lässt das Enzym entsprechend reagieren. Die Reaktion wird unmittelbar durch die bereits zuvor beschriebene optoelektrische Einheit ausgewertet. Im Anschluss wird das Gitternetz durch Kontakt mit Spülwasser gereinigt.

In einer weiteren bevorzugten Ausführungsform liegt der Indikator in Form von Rollen vor, von denen bei Analyse jeweils ein Bedarfsstück abgetrennt wird. Die Rollen befinden sich wie die bereits genannten Magazine in einem Gehäuse je nach Ausführungsform entweder auf der Innen- oder Außenseite der Toilette. Die Abtrennung des für die Messung notwendigen Indikatorstücks kann durch gängige Schneidmechanismen oder durch vorherige Präparation der Rolle passieren (beispielsweise Perforation zum Abreißen). Dadurch kann innerhalb der Vorrichtung einiger Platz gespart werden und zudem können mehrere Rollen darin vorgesehen werden. Es wäre auch möglich Indikatorrollen mit mehreren Indikationen auf einer Rolle zu nutzen.

In einer bevorzugten Ausführungsform wird für jeden Messvorgang ein einzelner Indikatorstreifen dem Gerät manuell zugeführt. Dies passiert über eine Trichteröffnung, einen Einzug (-sspalt) oder einen Einfuhrschieber, die an einer leicht zugänglichen Stelle am Gerät positioniert sind. Nach Zufuhr des Indikators wird diese vom Gerät zur Messposition im Inneren der Toilette transportiert und zweckmäßig positioniert. Der Transport zwischen Toilettenaußenseite und Sensorarm auf der Innenseite erfolgt entweder durch Schwerkraft, durch Unterdruck, durch (motorisierte) Rollen, Federkraft oder durch festeres Drücken des Nutzers. Auf der Innenseite erfolgt die Übergabe an den bereits mehrfach beschriebenen Sensorarm.

In einer weiteren bevorzugten Ausführungsform lassen sich die Indikatoreinheiten wieder über ein eingefügtes Magazin entsorgen. Dabei werden die Indikatoreinheiten nach Kontaktnahme mit Exkrement mittels des Sensorarms und anschließender Auswertung wieder in das Entsorgungsmagazin eingeführt. Dies kann entweder ein eigens dafür vorgesehenes Magazin sein oder ein Teil des Indikatorversorgungsmagazins, das durch die Entnahme einer Indikatoreinheit wiederum Platz für die Aufnahme der benutzten Indikatoreinheit bietet. Gebrauchte und ungebrauchte Indikatoreinheiten sind dabei voneinander getrennt. Das Entsorgungsmagazin kann einfach ausgetauscht werden.

Bei allen aufgeführten Indikatoren können sowohl Farb- als auch Musterunterschiede als Hinweis fungieren. Beispielsweise kann sich ein Indikator auch zusammenziehen, die Oberfläche wird rauer, es bildet sich eine Form heraus (beispielsweise zieht er sich zu einer Kugel zusammen) etc. Seitens der Auslesung ist es hier neben optoelektrischen Methoden auch möglich, über Schallwellentechnologie (beispielsweise Sonografie) die Werte zu bestimmen.

In einer alternativen Ausgestaltung ist der Indikator oder Sensor über eine in einer Führungshülse geführten Sensorarm in die Messposition bringbar. Auf diese Weise ist es möglich, dass der Sensorarm wesentlich dünner ausgeführt sein kann, was die Baugröße und Verschmutzungsmöglichkeiten deutlich reduziert.

Die Verschmutzungsbehebung oder Minimierung kann auch durch Rütteln bzw. Vibrieren des Sensorarms erreicht werden. Durch schnelle, ruckartige Bewegung des Sensorarms, vornehmlich hervorgerufen durch den Motor, kann Kontamination minimiert werden. Die Rüttelbewegung kann in unterschiedlichen Achsen erfolgen.

Eine weitere Variante, um die Verschmutzung des Sensormars zu verringern ist eine Abstreifvorrichtung für den Urin beim Zurückholen des Sensorarms. Dabei wird der Sensorarm mechanisch, chemisch oder elektrisch gereinigt. In einer weiteren bevorzugten Ausführungsform wird der Sensorarms beim Zurückfahren durch "Bürsten" von Exkrementen befreit. Die Bürsten sind so ausgestaltet, dass sie sich selbst reinigen oder regelmäßig ausgetauscht bzw. gewartet werden können. Grundsätzlich ist die Oberfläche des Sensormars so gestaltet, dass sich möglichst wenig Exkrement daran anhaftet. Dies passiert durch Geometrie und/oder Beschichtung. Bei speziell funktionalisierten Oberflächen kann bereits diese Maßnahme den gewünschten Effekt erzielen (bspw. Lotusblüteneffekt).

Um die Kontamination der Vorrichtung durch Exkrement zu minimalisieren wird in einer weiteren bevorzugten Ausführungsform eine Schutzhülle vorgesehen, die bei entsprechender Verschmutzung oder turnusmäßig ausgetauscht werden kann. Die Schutzhülle besteht vorzugsweise aus Kunststoff und kann über oder am Teil des Gerätes, der sich an der Innenseite der Toilette befindet, angebracht werden. Die Schutzhülle erstreckt sich vor allem auch über den Sensorarm. So kann bei zu starker Kontamination des Sensorarms schlicht die Schutzhülle entsorgt und eine neue angebracht werden.

Die Kalibration und Justage der Vorrichtung sowie des Sensorarms können in einer weiteren bevorzugten Ausführungsform unter Zuhilfenahme unterschiedlicher Informationsquellen vollzogen werden. Neben Daten zuvor beschriebener Sensoren (optoelektrisch oder sonografisch), die Standorte und relative Abstände zu Zielpositionen bestimmen, werden auch Informationen durch Kenntnis des Toilettenmodels oder Abgleich der grundsätzlichen Bauform genutzt. Die Rückmeldung an den Nutzer kann optisch oder akustisch erfolgen.

Ähnlich wird in einer weiteren bevorzugten Ausführungsform der jeweils optimale Ort zur Probenahme bestimmt. Zusätzlich wird dabei die Ausfahrlänge des Sensorarms sowie der Winkel, in dem sich der Indikator optimal an der Toilettenwand präsentiert, bestimmt. Neben Nutzung der zuvor beschriebenen Informationsquellen kann der Sensorarm zu diesem Zweck ausfahren und drucksensorische Erkennung ermöglichen. Dies passiert wenn der Arm beispielsweise die Toilettenwand berührt. Getroffene Einstellungen werden bei allen Kalibrationen und Justagen gespeichert, um eine wiederholte Anbringung beispielweise bei Abnahmen des Gerätes zur Reinigung der Toilette zu erleichtern. Nachkalibrierungen bei schlechten Probenahmen/Ergebnissen, ab einer gewissen Einsatzzeit oder -zahl sind ebenfalls möglich.

Eine weitere Lehre der Erfindung sieht vor, dass zur desinfizierenden Reinigung der Vorrichtung und/oder der gesamten Toilette eine UV-Lichtquelle vorgesehen ist. Diese Lichtquelle kann Teil der Vorrichtung sein oder aber auch nur mit dieser verbunden sein und einer anderen Stelle, beispielsweise auf der der Vorrichtung gegenüberliegenden inneren Seite der Toilettenschüssel angeordnet sein.

Die Erfindung wird nachfolgend anhand bevorzugter Ausführungsbeispiele näher erläutert. In der Zeichnung zeigen
- Fig. 1A und 1B: eine erste Ausführungsform der erfindungsgemäßen Vorrichtung, in perspektivischer Darstellung,
- Fig. 2 und 3: unterschiedliche Darstellungen der in den Fig. 1A und 1B gezeigten Ausführungsform der erfindungsgemäßen Vorrichtung. perspektivisch im Inneren einer Toilettenschüssel,
- Fig. 4: einen Vertikalschnitt durch die Vorrichtung aus Fig. 3 entlang der Linien IV-IV und
- Fig. 5: eine andere Ausführungsform der erfindungsgemäßen Vorrichtung, perspektivisch im Inneren einer Toilettenschüssel,
- Fig. 6: die Vorrichtung aus Fig. 5, perspektivisch von außen und
- Fig. 7: einen Teil der Vorrichtung aus Fig. 6, in perspektivischer Darstellung,
- Fig. 8: einen Horizontalschnitt durch den Gegenstand aus Fig. 7 entlang der Linie VIII-VIII,
- Fig. 9: eine weitere Ausführungsform der erfindungsgemäßen Vorrichtung.

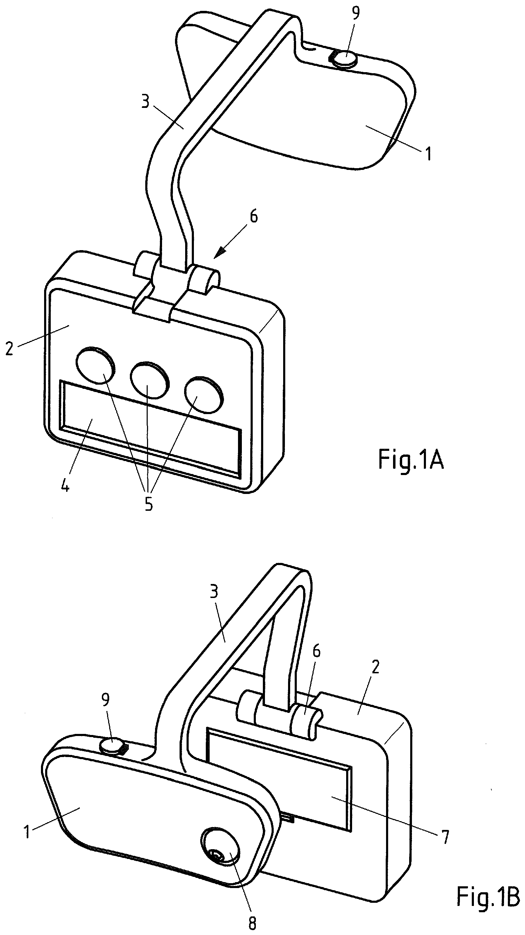

Die Figuren 1A und 1B zeigen ein Beispiel einer ersten Ausführungsform einer erfindungsgemäßen Vorrichtung in unterschiedlichen perspektivischen Darstellungen. Dabei weist die Vorrichtung neben einem Gehäuse 1 zur Aufnahme von (hier nicht dargestellten) Indikatoren auch eine Kontrolleinheit 2 auf. Im dargestellten und insoweit bevorzugten Ausführungsbeispiel sind das Gehäuse 1 und die Kontrolleinheit 2 mittels eines Haltebügels 3 miteinander verbunden. Der Haltebügel 3 ist eine besonders zweckmäßige Konstruktion, da er sowohl das Gehäuse 1 und die Kontrolleinheit 2 miteinander verbindet als auch zur Befestigung am Rand einer Toilettenschüssel genutzt werden kann. Es sind jedoch auch sämtliche anderen denkbaren Befestigungslösungen zur Anbringung der erfindungsgemäßen Vorrichtung wie Saugnäpfe, Klebe- oder ähnliche adhäsive Verbindungselemente von der Erfindung umfasst.

Die in Fig. 1A von der Bedienungsseite erkennbare Kontrolleinheit 2 weist ein Kontroll-Display 4, drei Bedienelemente 5 zum Auslösen und ggf. Einstellen der Vorrichtung sowie ein Gelenk 6 zur schwenkbaren Befestigung des Haltebügels 3 relativ zur Kontrolleinheit 2 auf. Dadurch kann das Kontroll-Display 4 zur besseren Bedienung durch den Benutzer verstellt werden.

Auf der in Fig. 1B erkennbaren Rückseite der Kontrolleinheit 2 ist eine Abdeckung 7 eines Fachs zur Aufnahme von Batterien oder Akkus erkennbar. Das Gehäuse 1 ist ferner mit einer Messeinheit 8 versehen, die beispielsweise als Kamera (optoelektrisch) oder/und sonografisch ausgeführt sein kann. Ein Magazin zur Aufnahme mehrerer Indikatorstreifen im Inneren des Gehäuses 1 ist mit einem Deckel 9 verschlossen. Das Gehäuse 1 kann auch noch über eine (nicht gezeigte) Lichtquelle verfügen.

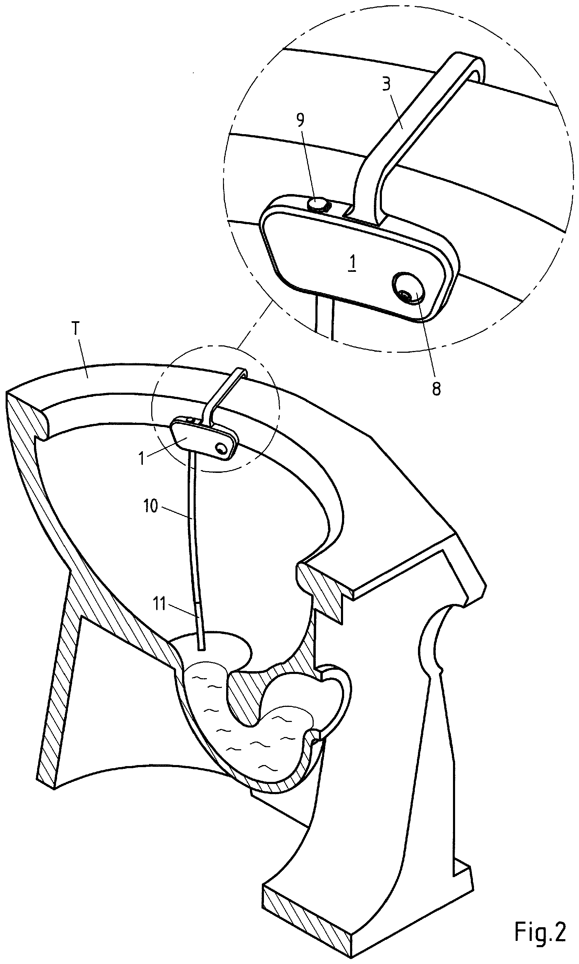

In Fig. 2 ist eine mögliche Anordnung der zuvor zu den Figuren 1A und 1B näher beschriebenen Vorrichtung in einer (zur besseren Übersicht aufgeschnitten dargestellten) standardisierten Tiefspültoilette T gezeigt, und zwar in einer perspektivischen Ansicht von schräg hinten. Man erkennt den Haltebügel 3 sowie das Gehäuse 1 mit Messeinheit 8 und Deckel 9 des Magazinfachs. Unterhalb des Gehäuses 1 sind ferner ein Sensorarm 10 und ein Indikator 11 zu erkennen. Am Ende des Sensorarms 10 ist der eigentliche Indikator 11 angeordnet, der nach Kontakt mit Exkrement und Auswertung durch die Messeinheit 8 über das Spülsystem der Toilette T entsorgt werden kann. Im dargestellten Ausführungsbeispiel ist der Sensorarm 10 aus dem Gehäuse 1 heraus teleskopisch ausfahrbar.

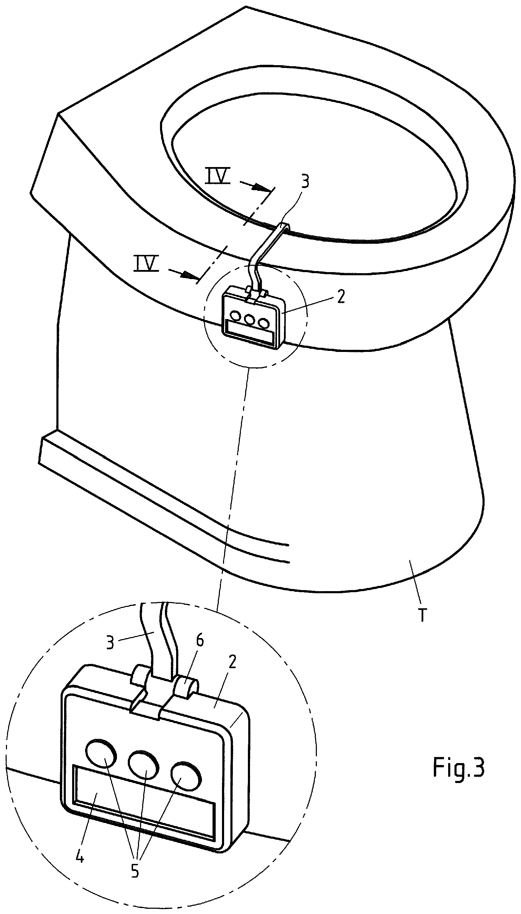

Fig. 3 zeigt die Vorrichtung aus den Figuren 1A und 2A in einer perspektivischen Außenansicht von schräg vorne. Neben dem Haltebügel 3 ist hier vor allem die Kontrolleinheit 2 mit dem Kontroll-Display 4 und den drei Bedienelementen 5 zum Auslösen und ggf. Einstellen des Messvorgangs zu erkennen. Man erkennt deutlich, dass die erfindungsgemäße Vorrichtung von außen kaum auffällt und leicht am Rand von Toilettenschüsseln (beliebiger Formen) angebracht werden kann.

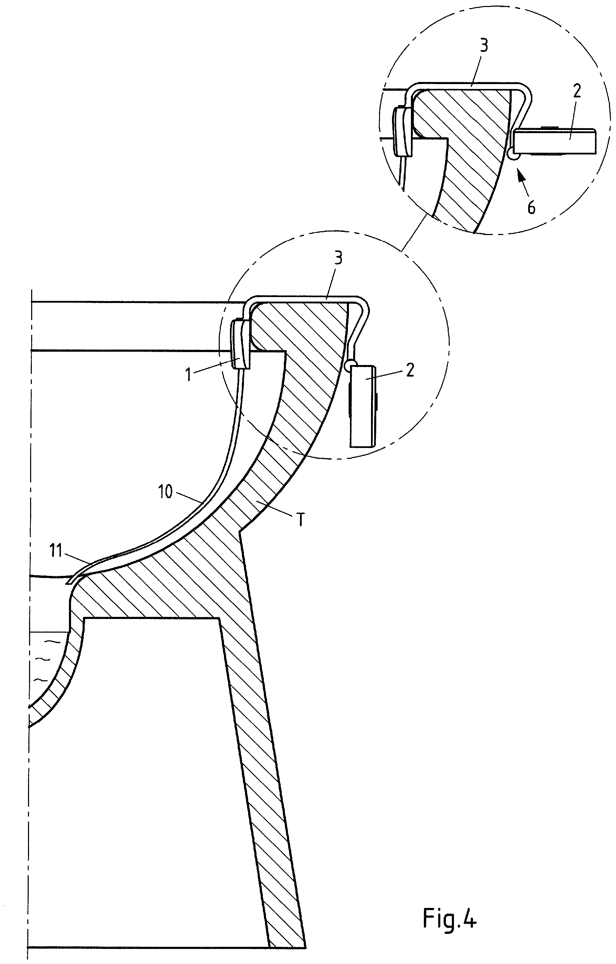

In Fig. 4 ist die erfindungsgemäße Vorrichtung mit Gehäuse 1, Haltebügel 3 und Kontrolleinheit 2 bei wiederum aufgeschnitten dargestellter Toilette T noch einmal in Ihrer Gesamtheit in einem Vertikalschnitt entlang der Linie IV-IV aus Fig. 3 dargestellt, zunächst mit heruntergeschwenkter Kontrolleinheit 2 und, rechts oben, mit um das Gelenk 6 hochgeschwenkter Kontrolleinheit 2.

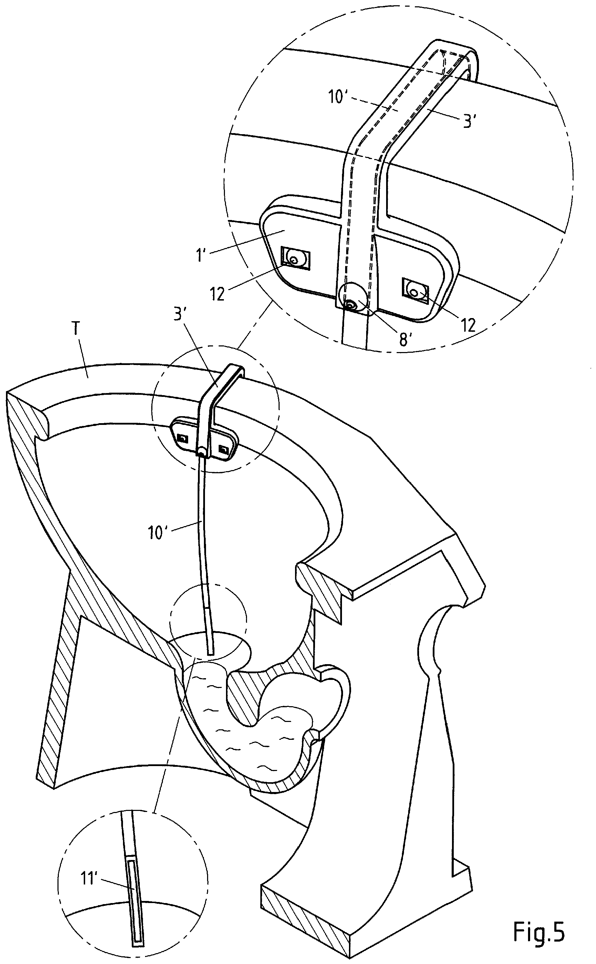

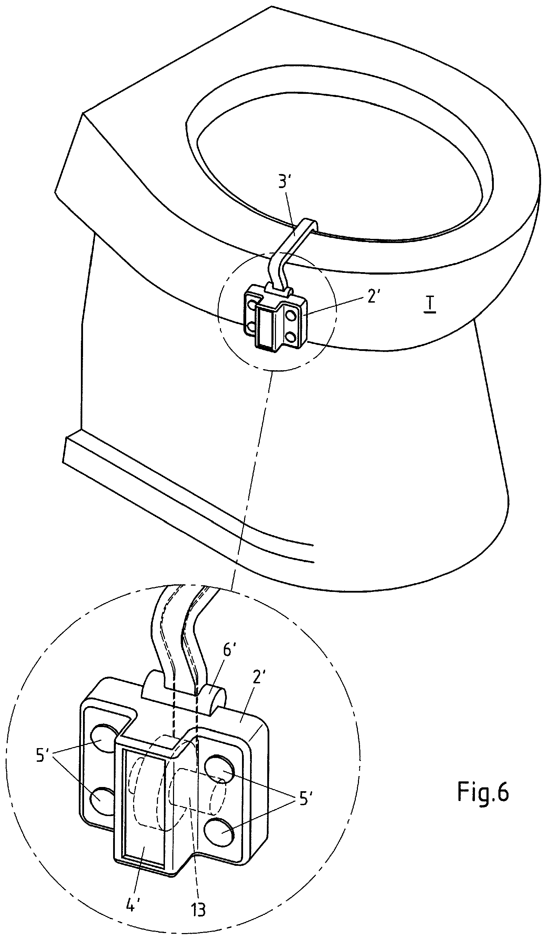

In den Fig. 5 und 6 ist ein anders bevorzugtes Ausführungsbeispiel der erfindungsgemäßen Vorrichtung in unterschiedlichen perspektivischen Darstellungen dargestellt. Die Messung erfolgt mittels eines Sensorarms 10', welcher an seinem Ende einen Indikator 11' aufweist. Die Vorrichtung weist ein Gehäuse 1' auf, welches im dargestellten und insoweit bevorzugten Ausführungsbeispiel mit einer Messeinheit 8' und Sensoren wie beispielsweise Kameras 12 versehen ist. Das Gehäuse 1' ist im Inneren einer Toilettenschüssel T angeordnet und mittels eines Bügels 3' mit einer Auswerte- und Kontrolleinheit 2' am äußeren Rand der Toilettenschüssel T verbunden, zweckmäßigerweise über eine Gelenkanordnung 6'. Der Bügel 3' kann dabei in seiner Länge verstellbar sein, um sich universal den verschiedenen Bauarten von Toilettenschüsseln anpassen zu können. Bei der dargestellten Ausführungsform wird der Sensorarm 10' von der Auswerte- und Kontrolleinheit 2' durch den Haltebügel 3' in das Gehäuse 1' im Toiletteninneren geführt.

Dies geschieht mit Hilfe eines in der Vergrößerung in Fig. 6 dargestellten Antriebs 13, der im dargestellten und insoweit bevorzugten Ausführungsbeispiel innerhalb der Auswerte- und Kontrolleinheit 2' angeordnet ist. Der Antrieb könnte auch im Gehäuse 1' angeordnet sein. Der Sensorarm 10' liegt im eingefahrenen Zustand aufgerollt vor und entrollt sich beim Ausfahren mit Hilfe des Antriebs 13. Beim Verlassen des Gehäuses 1' im Toiletteninneren versteift sich der Sensorarm 10' wie in Fig. 6 dargestellt und kann somit zielgerichtet an den Ort der Messung geführt werden. Die Versteifung erfolgt durch Materialeigenschaften und/oder durch mechanische Verformung. Zur Messung dient die Messeinheit 8', welche bei dieser Ausführungsform mittig über dem Auslass des Sensorarms 10' angeordnet ist, um den Indikator 11' optimal auswerten zu können. Links und rechts davon sind zwei Zeilenkameras 12 angeordnet, mit deren Hilfe einerseits der Messzeitpunkt bestimmt und andererseits eine Volumenstrommessung und/oder eine Massebestimmung des Exkrements vorgenommen werden kann.

Die Auswerte- und Kontrolleinheit 2' verfügt ferner über ein Kontroll-Display 4' und eine Mehrzahl von Bedienelementen 5' zur Durchführung der Messung und Auswertung.

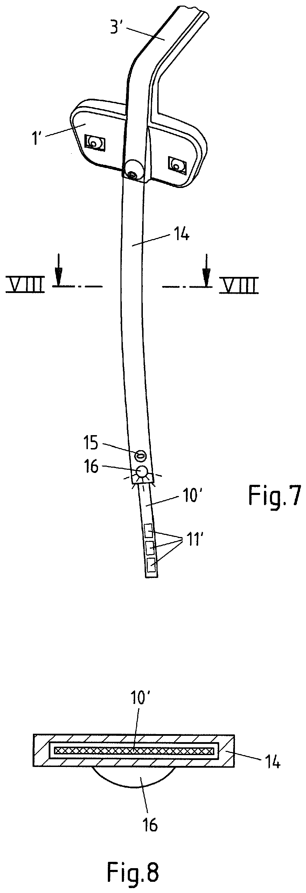

Eine alternative Ausführungsform ist in Fig. 7 gezeigt. Dabei wird der Sensorarm 10' im Inneren der Toilette durch eine hohle Führungshülse 14 so weit ins Toiletteninnere zwangsgeführt, bis der Sensorarm 10' mit darauf befindlichem/n Indikator/en 11' die Messposition nahezu erreicht hat. Diese Ausführungsform kann somit auf eine selbstversteifende Ausführung des Sensorarmes verzichten. Am unteren Ende der Führungshülse 14 sind im dargestellten und insoweit bevorzugten Ausführungsbeispiel ein Sensor 15 und ein Leuchtmittel 16 angebracht, deren Anschlussleitungen ebenfalls an oder bevorzugt in der Führungshülse 14 angeordnet sind.

Fig. 8 zeigt einen Schnitt durch die Führungshülse 14 entlang der Linie VIII-VIII in Fig. 7. Man erkennt deutlich, dass der Sensorarm 10' bei dieser Ausführung sehr dünn ausgebildet sein kann. Bei dem in Fig. 7 dargestellten Ausführungsbeispiel ist gezeigt, dass anstelle eines einzigen Indikators auch mehrere unterschiedliche Indikatoren 11' zum Einsatz kommen können

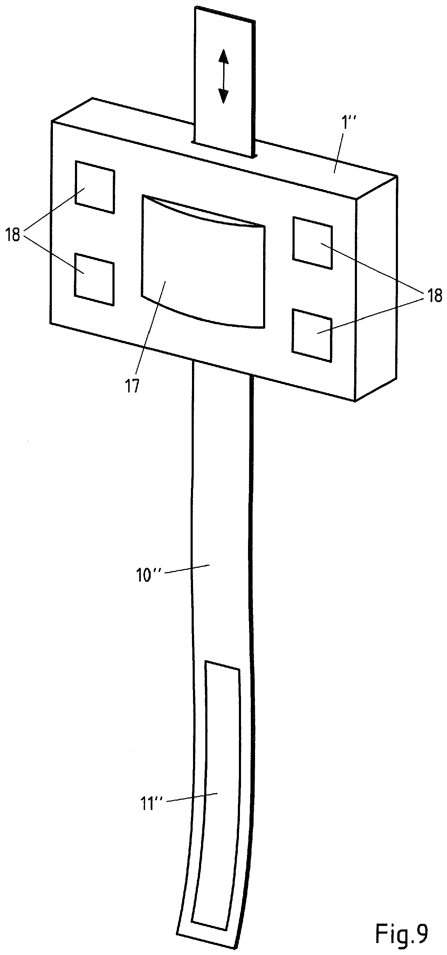

Fig. 9 zeigt schließlich schematisch einen Grundaufbau einer weiteren Ausführungsform der erfindungsgemäßen Vorrichtung in ihrer einfachsten und rudimentärsten Bauart. Diese besteht zunächst und im Wesentlichen aus einem Sensorarm 10", der an seinem unteren Ende einen Indikator 11" aufweist und einem Gehäuse 1" zum Halten, Darbieten und Benutzen des Sensorarms 10". Im dargestellten und bevorzugten Ausführungsbeispiel verfügt das Gehäuse 1" über ein Sichtfenster 17 zum Vergleich und ggf. zur Vergrößerung eines Messergebnisses mit Normparametern, welche - beispielsweise als Farbmarkierungen 18 - für einen einfachen optischen Vergleich unmittelbar auf dem Gehäuse 1" neben dem Sichtfenster 17 angeordnet sein können.

Der Sensorarm 10" ist relativ zum Gehäuse 1" in Richtung entlang des Doppelpfeils bewegbar und wird zur optischen Auswertungen vom Ort des Exkrement-Kontakts innerhalb der Toilette manuell so weit zurückgeholt, dass der Benutzer den eigentlichen Indikatorbereich gut und einfach ablesen kann. Das Gehäuse 1" kann dabei mit geeigneten Befestigungsvorrichtungen (nicht dargestellt) wie beispielsweise Saugnäpfen od. dgl. am oberen Rand einer (nicht dargestellten) Toilettenschüssel befestigt werden.

## Patentansprüche

1. Vorrichtung zur Bestimmung physiologischer Daten durch Analyse menschlicher Exkremente in einer Toilette (T) mittels wenigstens einem Indikator (11, 11', 11") und/oder Sensor, umfassend ein Messsystem, bei dem für jede Messung ein Indikator (11, 11', 11") bzw. Sensor in eine Messposition bringbar ist, in der ein ausreichender Kontakt mit dem zu untersuchenden Urin oder Stuhl erfolgt, wobei der Indikator (11, 11', 11") bzw. Sensor als eigenständige Einheit ausgebildet ist/sind,
**gekennzeichnet dadurch, dass** der eigentliche Indikator (11, 11', 11") bzw. Sensor mittels eines ausrollbaren oder eines teleskopisch ausfahrbaren Elements in die Messposition ins Toiletteninnere bringbar ist.

2. Vorrichtung nach Anspruch 1, ***dadurch gekennzeichnet, dass*** der eigentliche Indikator (11, 11', 11") bzw. Sensor von der Außenseite der Toilette (T) mittels des ausrollbaren Elements ins Toiletteninnere bringbar ist.

3. Vorrichtung zur Bestimmung physiologischer Daten durch Analyse menschlicher Exkremente in einer Toilette (T) mittels wenigstens einem Indikator (11, 11', 11") und/oder Sensor, umfassend ein Messsystem, bei dem für jede Messung ein Indikator (11, 11', 11") bzw. Sensor in eine Messposition bringbar ist, in der ein ausreichender Kontakt mit dem zu untersuchenden Urin oder Stuhl erfolgt, wobei der Indikator (11, 11', 11") bzw. Sensor als eigenständige Einheit ausgebildet ist/sind,
**gekennzeichnet dadurch, dass** der eigentliche Indikator (11, 11', 11") bzw. Sensor von der Außenseite der Toilette (T) mittels eines bewegbaren Elements in die Messposition ins Toiletteninnere bringbar ist.

4. Vorrichtung nach einem der Ansprüche 1 bis 3, ***dadurch gekennzeichnet, dass*** der eigentliche Indikator (11, 11', 11") bzw. Sensor mittels eines wenigstens teilweise selbstversteifenden Sensorarms (10, 10', 10") in die Messposition bringbar ist.

5. Vorrichtung nach einem der Ansprüche 1 bis 4, ***dadurch gekennzeichnet, dass*** ein oder mehrere Indikatoren (11, 11', 11") bzw. Sensoren in einem Gehäuse (1, 1', 1") angeordnet sind und wobei das Gehäuse an der Toilettenschüssel reversibel befestigbar ist.

6. Vorrichtung nach einem der Ansprüche 1 bis 5, ***dadurch gekennzeichnet, dass*** der wenigstens eine Indikator (11, 11', 11") bzw. Sensor als Einmalindikator oder Einmalsensor ausgebildet ist und bevorzugt durch die Spülung entsorgt wird.

7. Vorrichtung nach einem der Ansprüche 1 bis 5, ***dadurch gekennzeichnet, dass*** der wenigstens eine Indikator (11, 11', 11") bzw. Sensor als Mehrfachindikator oder Mehrfachsensor ausgebildet ist.

8. Vorrichtung nach einem der Ansprüche 1 bis 7, ***dadurch gekennzeichnet, dass*** eine Mehrzahl von Indikatoren (11, 11', 11") bzw. Sensoren wenigstens ein nachfüllbares Magazin vorhanden ist.

9. Vorrichtung nach einem der Ansprüche 1 bis 8, ***dadurch gekennzeichnet, dass*** Indikator bzw. Sensorarm durch eine Reinigungsvorrichtung von überflüssigem Exkrement befreit werden.

10. Vorrichtung nach einem der Ansprüche 1 bis 9, ***dadurch gekennzeichnet, dass*** zur Desinfektion des Sensorarms, der Vorrichtung und/oder des Messortes eine UV-Lichtquelle vorgesehen ist.

11. Vorrichtung nach einem der Ansprüche 1 bis 10, ***dadurch gekennzeichnet, dass*** die Vorrichtung wenigstens eine optoelektrische Einheit und/oder eine sonografische Einheit umfasst.

12. Vorrichtung nach einem der Ansprüche 1 bis 11, ***dadurch gekennzeichnet, dass*** die Vorrichtung eine Auswerteeinrichtung, eine Kommunikationseinrichtung und/oder eine Analysevorrichtung umfasst.

13. Verfahren zur Bestimmung physiologischer Daten durch Analyse menschlicher Exkremente in einer Toilette (T) durch Messung von Urin- und/oder Stuhlwerten, mittels einer Vorrichtung nach einem der Ansprüche 1 bis 12, ***dadurch gekennzeichnet, dass*** eine Mehrzahl zuvor bestimmter Werte gemessen und anschließend, bevorzugt automatisiert, weiterverarbeitet und ggf. weitergeleitet wird.

14. Verfahren nach Anspruch 13, ***dadurch gekennzeichnet, dass*** mit Hilfe von optoelektrischen Einheiten durch stereoskopische Aufnahmen Exkrementmenge und -volumen sowie Exkrementvolumen- bzw. -massenstrom bestimmt werden.

15. Verfahren nach Anspruch 13 oder 14, ***dadurch gekennzeichnet, dass*** der Indikator oder Sensor in flüssiger Form in der Vorrichtung vorliegt und über Versorgungselemente, wie beispielsweise ein Gitternetz oder eine Matrix, mit der Probe bzw. Exkrementen in Kontakt gebracht wird.

16. Verfahren nach einem der Ansprüche 13 bis 15, ***dadurch gekennzeichnet, dass*** eine Verlaufsbetrachtung der Indikatorreaktion über eine bestimmte Zeitdauer erfolgt und daraus zusätzliche Informationen generiert werden.

17. Verfahren nach einem der Ansprüche 13 bis 16, ***dadurch gekennzeichnet, dass*** der optimale Messzeitpunkt durch die Vorrichtung mittels einzelner Sensoren oder einer Kombination aus Wärmesensoren, optischer Auswertung und Akustiksensoren bestimmt wird.

18. Verfahren nach einem der Ansprüche 13 bis 17, ***dadurch gekennzeichnet, dass*** zur optimalen Anbringung der Vorrichtung an der Toilette sowie der Ausrichtung in der Toilette eine automatische oder geführte Kalibrierung und/oder Justierung erfolgt.

19. Verfahren nach einem der Ansprüche 13 bis 18, ***dadurch gekennzeichnet, dass*** die Vorrichtung zur Identifikation einer bestimmten Person, beispielsweise ein Benutzer, verwendet wird und nach der Messung eine Ergänzung der gewonnenen Messwerte mit weiteren Vital- und Personenüberwachungsparametem erfolgt.

20. Verfahren nach einem der Ansprüche 13 bis 19, ***dadurch gekennzeichnet, dass*** die Messung und/oder Weiterverarbeitung der Messwerte automatisch erfolgt und die Werte an eine Auswerteeinrichtung, beispielsweise ein Smartphone, Tablet, Wearable oder sonstiges, mobiles oder stationäres Endgerät gesendet und dort gespeichert, weiterverarbeitet oder weitergeleitet werden.

21. Nicht-therapeutische bzw. nicht-diagnostische Verwendung der Vorrichtung nach einem der Ansprüche 1 bis 12 bzw. des Verfahrens nach einem der Ansprüche 13 bis 20 zur Bestimmung eines oder mehrerer der Nachfolgend genannten Untersuchungen:
- Bestimmung des Zeitpunktes im Menstruationszyklus,
- Bestimmung des Blutalkoholwertes,
- Nachweis einer Schwangerschaft,
- Nachweis von Drogenkonsum,
- (früh-)Erkennung von gutartigen (benignen) bzw. bösartigen (malignen) Tumoren,
- Überwachung von Diäten,
- Kontrolle des Stoffwechsels,
- Kontrolle von Medikationen und
- Kontrolle der Standard-Urinwerte ("Urin-Schnelltest").

## Claims

1. Device for determining physiological data by analyzing human excrements in a toilet (T), using at least one indicator (11, 11', 11") and/or sensor, comprising a measurement system where for each measurement, an indicator (11, 11', 11") or sensor, respectively, can be brought in a measurement position in which sufficient contact with the urine or stool to be examined is established, the indicator (11, 11', 11") or sensor, respectively, being embodied as an independent unit; ***characterized in that*** the actual indicator (11, 11', 11") or sensor, respectively, can be placed in the measurement position inside the toilet by means of an element which can be unrolled or extended telescopically.

2. Device according to Claim 1, ***characterized in* that** the actual indicator (11, 11', 11") or sensor, respectively, can be positioned inside the toilet from the outside of the toilet (T) by means of the element which can be unrolled.

3. Device for determining physiological data by analyzing human excrements in a toilet (T), using at least one indicator (11, 11', 11") and/or sensor, comprising a measurement system where for each measurement, an indicator (11, 11', 11") or sensor, respectively, can be brought in a measurement position in which sufficient contact with the urine or stool to be examined is established, the indicator (11, 11', 11") or sensor, respectively, being embodied as an independent unit; ***characterized in* that** the actual indicator (11, 11', 11") or sensor, respectively, can be placed in the measurement position inside the toilet by means of a movable element from the outside of the toilet (T).

4. Device according to one of Claims 1 through 3, **characterized in that** the actual indicator (11, 11', 11") or sensor, respectively, can be placed in the measurement position by means of an at least partly self-reinforcing sensor arm (10, 10', 10").

5. Device according to one of Claims 1 through 4, ***characterized* in that** one or more indicators (11, 11', 11") or sensors, respectively, are arranged in a housing (1, 1', 1"), the housing being reversibly attachable in the toilet bowl.

6. Device according to one of Claims 1 through 5, ***characterized* in that** the at least one indicator (11, 11', 11") or sensor, respectively, is embodied as a disposable indicator or disposable sensor and is preferably disposed of by flushing.

7. Device according to one of Claims 1 through 5, ***characterized* in that** the at least one indicator (11, 11', 11") or sensor, respectively, is embodied as a reusable indicator or reusable sensor.

8. Device according to one of Claims 1 through 7, ***characterized* in that** there is a plurality of indicators (11, 11', 11") or sensors, respectively, at least one refillable magazine.

9. Device according to one of Claims 1 through 8, ***characterized* in that** the indicator or sensor arm, respectively, are cleaned of excess excrements by a cleaning device.

10. Device according to one of Claims 1 through 9, ***characterized* in that** for disinfection of the sensor arm, the device and/or the measuring site, a UV light source is provided.

11. Device according to one of Claims 1 through 10, ***characterized* in that** the device comprises at least one optoelectronic unit and/or a sonographic unit.

12. Device according to one of Claims 1 through 11, ***characterized* in that** the device comprises an evaluation unit, a communication unit and/or an analyzing unit.

13. Method of determining physiological data by analyzing human excrements in a toilet (T) by measuring urine and/or stool values by means of a device according to one of Claims 1 through 12, ***characterized in* that** a plurality of predefined values is measured and subsequently processed and, if desired, relayed, preferably in an automated manner.

14. Method according to Claim 13, ***characterized in* that** by means of optoelectric units, the amount and volume of excrement as well as the excrement volume flow and excrement mass flow rate, respectively, are determined using stereoscopic images.

15. Method according to Claim 13 or 14, ***characterized in* that** the indicator or sensor is present in the device in liquid form and is brought in contact with the sample or the excrements, respectively, by means of provision elements such as, for example, a grid or a matrix.

16. Method according to one of Claims 13 through 15, ***characterized in* that** the progression of the indicator reaction is monitored over a specific duration of time and additional information is generated from it.

17. Method according to one of Claims 13 through 16, ***characterized in* that** the optimum point in time for measurement is determined by the device by means of individual sensors or a combination of heat sensors, optical evaluation and acoustic sensors.

18. Method according to one of Claims 13 through 17, ***characterized in* that** for optimum attachment of the device to the toilet and adjustment in the toilet, an automated or controlled calibration and/or adjustment takes place.

19. Method according to one of Claims 13 through 18, ***characterized in* that** the device is used to identify a specific person, for instance a user, and after measurement the measured values are supplemented with additional vital and individual monitoring parameters.

20. Method according to one of Claims 13 through 19, ***characterized in* that** the measurement and/or processing of the measured values takes place in an automated manner and the values are transmitted to an evaluation unit, for instance a smartphone, tablet, a wearable device or a different type of mobile or stationary terminal and are stored, processed or relayed there.

21. Non-therapeutic or non-diagnostic use, respectively, of the device according to one of Claims 1 through 12 or of the method according to one of Claims 13 through 20, respectively, for determining one or more of the following analyses:
- determining the point in time of the menstruation cycle,
- determining the blood alcohol level,
- detection of pregnancy,
- detection of drug consumption,
- (early) detection of benign or malign tumors,
- diet monitoring,
- monitoring of metabolism,
- monitoring of medication and
- monitoring of the standard urine values (rapid analysis of urine).

## Revendications

1. Dispositif destine à la détermination de données physiologiques par l'analyse d'excréments humains dans des toilettes (T) au moyen d'au moins un indicateur (11, 11', 11") et/ou d'un capteur, comprenant un système de mesure, pour lequel pour chaque mesure un indicateur (11, 11', 11") ou un capteur peut être placé dans une position de mesure dans laquelle un contact suffisant avec l'urine ou les selles à analyser est établi, sachant que l'indicateur (11, 11', 11") ou le capteur est/sont constitué(s) sous la forme d'une unité propre, **caractérisé en ce que** l'indicateur (11, 11', 11") ou le capteur proprement dit peut être placé dans la position de mesure à l'intérieur des toilettes au moyen d'un élément déroulable ou amovible de façon télescopique.

2. Dispositif selon la revendication 1, **caractérisé en ce que** l'indicateur (11, 11', 11") ou le capteur proprement dit peut être placé depuis le côté extérieur des toilettes (T) à l'intérieur des toilettes au moyen de l'élément déroulable.

3. Dispositif destiné à la détermination de données physiologiques par l'analyse d'excréments humains dans des toilettes (T) au moyen d'au moins un indicateur (11, 11', 11") et/ou d'un capteur, comprenant un système de mesure, pour lequel pour chaque mesure un indicateur (11, 11', 11") ou un capteur peut être placé dans une position de mesure dans laquelle un contact suffisant avec l'urine ou les selles à analyser est établi, sachant que l'indicateur (11, 11', 11") ou le capteur est/sont constitué(s) sous la forme d'une unité propre, **caractérisé en ce que** l'indicateur (11, 11', 11") ou le capteur proprement dit peut être placé depuis le côté extérieur des toilettes (T) dans la position de mesure à l'intérieur des toilettes au moyen d'un élément mobile.

4. Dispositif selon l'une quelconque des revendications 1 à 3, **caractérisé en ce que** l'indicateur (11, 11', 11") ou le capteur proprement dit peut être placé dans la position de mesure au moyen d'un bras de capteur (10, 10', 10") au moins en partie autoraidissant.

5. Dispositif selon l'une quelconque des revendications 1 à 4, **caractérisé en ce qu'**un ou plusieurs indicateurs (11, 11', 11") ou capteurs sont disposés dans un boîtier (1, 1', 1") et sachant que le boîtier peut être fixé de façon réversible à la cuvette des toilettes.

6. Dispositif selon l'une quelconque des revendications 1 à 5, **caractérisé en ce qu'**au moins un indicateur (11, 11', 11") ou un capteur est constitué sous la forme d'un indicateur à usage unique ou d'un capteur à usage unique et est de préférence éliminé dans la chasse d'eau.

7. Dispositif selon l'une quelconque des revendications 1 à 5, **caractérisé en ce qu'**au moins un indicateur (11, 11', 11") ou un capteur, est constitué sous la forme d'un indicateur multiple ou d'un capteur multiple.

8. Dispositif selon l'une quelconque des revendications 1 à 7, **caractérisé en ce qu'**il y a une pluralité d'indicateurs (11, 11', 11") ou de capteurs, au moins un magasin pouvant être rempli.

9. Dispositif selon l'une quelconque des revendications 1 à 8, **caractérisé en ce que** l'indicateur ou le bras de capteur sont libérés des excréments superflus par un dispositif de nettoyage.

10. Dispositif selon l'une quelconque des revendications 1 à 9, **caractérisé en ce qu'**une source de lumière UV est prévue pour la désinfection du bras de capteur, du dispositif et/ou du site de mesure.

11. Dispositif selon l'une quelconque des revendications 1 à 10, **caractérisé en ce que** le dispositif comprend au moins une unité opto-électrique et/ou une unité sonographique.

12. Dispositif selon l'une quelconque des revendications 1 à 11, **caractérisé en ce que** le dispositif comprend un système d'exploitation, un système de télécommunication et/ou un dispositif d'analyse.

13. Procédé destiné à la détermination de données physiologiques par analyse des excréments humains dans des toilettes (T) par la mesure des valeurs relatives à l'urine et/ou aux selles, au moyen d'un dispositif selon l'une quelconque des revendications 1 à 12, **caractérisé en ce qu'**une pluralité de valeurs préalablement déterminées sont mesurées et ensuite de préférence automatisées, ultérieurement traitées et le cas échéant transmises.

14. Procédé selon la revendication 13, **caractérisé en ce que** la quantité et le volume d'excréments ainsi que le débit volumique ou massique d'excréments sont déterminés à l'aide d'unités opto-électriques par des enregistrements stéréoscopiques.

15. Procédé selon la revendication 13 ou 14, **caractérisé en ce que** l'indicateur ou le capteur existe sous une forme liquide dans le dispositif et est mis en contact avec l'échantillon ou les excréments par le biais d'éléments d'alimentation, comme par exemple un réseau de grille ou une matrice.

16. Procédé selon l'une quelconque des revendications 13 à 15, **caractérisé en ce qu'**une considération de l'évolution de la réaction d'indicateur a lieu sur une période de temps définie et que des informations supplémentaires sont générées à partir de celle-ci.

17. Procédé selon l'une quelconque des revendications 13 à 16, **caractérisé en ce que** le moment de mesure optimal par le dispositif est déterminé au moyen de capteurs individuels ou d'une combinaison de capteurs thermiques, d'évaluation optique et de capteurs acoustiques.

18. Procédé selon l'une quelconque des revendications 13 à 17, **caractérisé en ce qu'**un étalonnage et/ou ajustement automatique ou guidé a lieu pour la mise en place optimale du dispositif sur les toilettes ainsi que pour l'orientation dans les toilettes.

19. Procédé selon l'une quelconque des revendications 13 à 18, **caractérisé en ce que** le dispositif est utilisé pour l'identification d'une personne définie, par exemple un utilisateur et après la mesure un complément des valeurs de mesure acquises a lieu avec d'autres paramètres de contrôle vitaux et personnels.

20. Procédé selon l'une quelconque des revendications 13 à 19, **caractérisé en ce que** la mesure et/ou le traitement ultérieur des valeurs de mesure a lieu automatiquement et les valeurs sont envoyées et mémorisées, traitées ultérieurement ou transmises à un système d'exploitation, par exemple, un téléphone intelligent, une tablette, un appareil portable, ou autre terminal mobile ou fixe.

21. Utilisation non-thérapeutique ou non diagnostique du dispositif selon l'une quelconque des revendications 1 à 12 ou du procédé selon l'une quelconque des revendications 13 à 20 destiné à la détermination d'une ou de plusieurs analyses citées ci-après :
- détermination de la période dans le cycle de menstruation,
- détermination du taux d'alcool sanguin
- décèlement d'une grossesse,
- décèlement de la consommation de stupéfiants
- identification précoce de tumeurs bénignes ou malignes,
- surveillance des régimes,
- contrôle du métabolisme,
- contrôle des médications, et
- contrôle des valeurs urinaires standard (test urinaire rapide)
